(19) Europäisches Patentamt
European Patent Office
Office européen des brevets

(11) Publication number: 0 388 164 A1

(12) # EUROPEAN PATENT APPLICATION

(21) Application number: 90302693.8

(22) Date of filing: 14.03.90

(51) Int. Cl.5: C07D 493/08, C07D 405/12, C07D 413/12, C07D 417/12, A01N 43/90, A01N 43/40, A01N 43/56, A01N 43/653, A01N 43/76, A01N 43/78

(30) Priority: 14.03.89 US 323574
28.02.90 US 484087

(43) Date of publication of application:
19.09.90 Bulletin 90/38

(84) Designated Contracting States:
GR

(71) Applicant: E.I. DU PONT DE NEMOURS AND COMPANY
1007 Market Street
Wilmington Delaware 19898(US)

(72) Inventor: Bozarth, Gene Allen
15 Nathalie Drive
Hockessin, Delaware 19707(US)
Inventor: Christensen, Joel Robert
36 Choate Street
Newark, Delaware 19711(US)
Inventor: Powell, James Edward
168 Chandlee Road
Rising Sun, Maryland 21911(US)
Inventor: Schlecht, Matthew Fred
18 West Ridge Court
Newark, Delaware 19711(US)

(74) Representative: Hildyard, Edward Martin et al
Frank B. Dehn & Co. European Patent Attorneys Imperial House 15-19 Kingsway
London WC2B 6UZ(GB)

(54) **Selective oxabicycloalkanes.**

(57) This invention relates to compounds, agriculturally suitable compositions thereof, and a method for controlling the growth of undesired vegetation in paddy rice with an oxabicycloalkane herbicide of the formula
$JOCH_2Q$
wherein J is an oxabicycloalkane moiety; and
Q is an optionally substituted aromatic or heteroaromatic moiety.

EP 0 388 164 A1

# SELECTIVE OXABICYCLOALKANES

## Background of the Invention

This invention relates to compounds, agriculturally suitable compositions thereof, and a method for controlling the growth of undesired vegetation in paddy rice with an oxabicycloalkane herbicide.

U.S. 4,670,041 and U.S. 4,486,219 disclose oxabicycloalkanes and their use for controlling plant growth.

U.S. 4,798,621 discloses [2.2.1]oxabicycloethers for the control of weeds in rice.

## Summary of the Invention

This invention comprises a method for controlling the growth of undesired vegetation in a paddy rice crop by applying to the locus of the paddy rice crop an effective amount of a compound of Formula I

$JOCH_2Q$ Formula I

wherein

J is

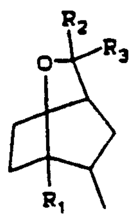 , 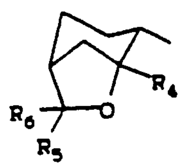 or 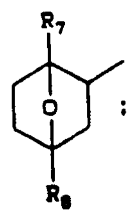 ;

J-1 J-2 J-3

$R_1$ is $CH_3$ or $CH_2CH_3$;

$R_2$ and $R_3$ are independently H, $CH_3$ or $CH_2CH_3$ or

$R_2$ and $R_3$ may be taken together as $-(CH_2)n-$where n is 4 or 5;

$R_4$ is H, $CH_3$ or $CH_2CH_3$;

$R_5$ and $R_6$ are independently $CH_3$ or $CH_2CH_3$;

$R_7$ is $CH_3$ or $CH_2CH_3$;

$R_8$ is $CH_3$, $CH_2CH_3$ or $CH(CH_3)_2$;

Q is

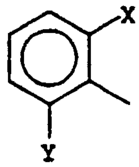 , 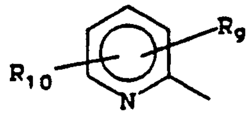 ,

Q-1 Q-2

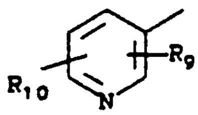 , 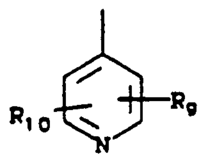 , 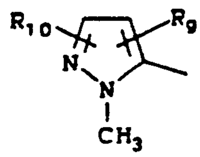 ,

Q-3 Q-4 Q-5

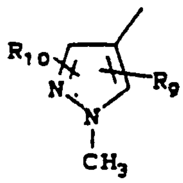 , 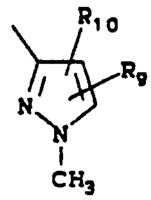 , 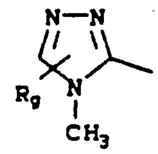

Q-6 Q-7 Q-8

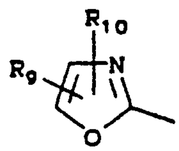 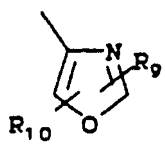 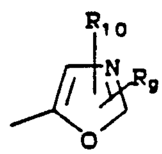

Q-9 Q-10 Q-11

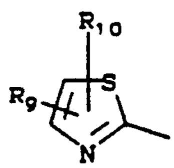 , 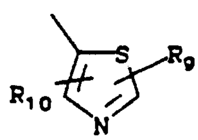 or 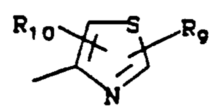

Q-12 Q-13 Q-14

X is F, Cl, or $CH_3$;
Y is H, F, or Cl;
$R_9$ is H, F, Cl, Br, $CH_3$ or $CH_2CH_3$; and
$R_{10}$ is H, F, Cl or $CH_3$;
provided that

1. when J is J-1 or J-3 and Q is Q-2 then $R_9$ and $R_{10}$ are not both H.
2. when Q is Q-1 then J is J-1, $R_1$ is $CH_3$, and $R_2$ and $R_3$ are both $CH_2CH_3$.
3. when X is F then Y is other than H.
4. when X is Cl then Y is other than Cl.
5. when X is $CH_3$ then Y is H.
6. when Y is H then X is Cl or $CH_3$.
7. when Y is F then X is F or Cl.
8. when Y is Cl then X is F or H.

Preferred for reasons of more efficient weed control and/or better crop tolerance are:

1. The method wherein

$R_1$ is $CH_3$;
$R_2$ and $R_3$ are $CH_2CH_3$ or $R_2$ and $R_3$ are taken together as $-(CH_2)n-$ where n is 4 or 5;

$R_4$ is $CH_3$;
$R_5$ and $R_6$ are $CH_3$;
$R_7$ is $CH_3$ or $CH_2CH_3$; and
$R_8$ is $CH_2CH_3$ or $CH(CH_3)_2$.

2. The method of Preferred 1 wherein J is J-1.
3. The method of Preferred 2 wherein Q is Q-1.
4. The method of Preferred 2 wherein Q is Q-2.
5. The method of Preferred 2 wherein Q is Q-3 or Q-4.
6. The method of Preferred 2 wherein Q is Q-5 through Q-14.
7. The method of Preferred 1 wherein J is J-2.
8. The method of Preferred 7 wherein Q is Q-2.
9. The method of Preferred 7 wherein Q is Q-3 or Q-4.
10. The method of Preferred 7 wherein Q is Q-5 through Q-14.
11. The method of Preferred 1 wherein J is J-3.
12. The method of Preferred 12 wherein Q is Q-2.
13. The method of Preferred 12 wherein Q is Q-3.
14. The method of Preferred 12 wherein Q is Q-4.
15. The method of Preferred 12 wherein Q is Q-5 or Q-6.
16. The method of Preferred 12 wherein Q is Q-7 through Q-14.
17. The method of Preferred 1 wherein the crop is transplanted japonica rice.
18. The method of Preferred 1 wherein the crop is transplanted indica rice.
19. The method of Preferred 1 wherein among the weeds controlled is barnyardgrass.

Specifically preferred for reasons of most efficient weed control and/or better crop tolerance is the method wherein the compound of Formula I is selected from the group consisting of:

6-endo-[(2,6-difluorophenyl)methoxy]-3,3-diethyl-1-methyl-2-oxabicyclo[2.2.2]octane;
6-endo-[(2-chlorophenyl)methoxy]-3,3-diethyl-1-methyl-2-oxabicyclo-[2.2.2]octane;
6-endo-[(2-chloro-6-fluoro phenyl)methoxy]-3,3-diethyl-1-methyl-2-oxabicyclo[2.2.2.]octane;
3,3-diethyl-1-methyl-6-endo-[(2-methylphenyl)methoxy]-2-oxabicyclo[2.2.2]octane;
exo-1-methyl-4-(1-methylethyl)-2-[(4′-thiazolyl)methoxy]-7-oxabicyclo[2.2.1]heptane;
exo-1,4-diethyl-2-[(4′-thiazolyl)methoxy]-7-oxabicyclo[2.2.1]heptane;
exo-1-methyl-4-(1-methylethyl)-2-[(2′-(6′-fluoropyridyl))methoxy]-7-oxabicyclo[2.2.1]heptane; and
exo-1,4-diethyl-2-[(2′-(6′-fluoropyridyl))methoxy]-7-oxabicyclo[2.2.1]heptane.

This invention also comprises compounds of Formula I and agriculturally suitable compositions containing them wherein

Q is Q-2 through Q-14,
provided that when J is J-1 or J-3 and Q is Q-2 then $R_9$ and $R_{10}$ are not both H.

Preferred for reasons of more efficient weed control and/or better crop tolerance are:

1. Compound of the Formula I wherein

$R_1$ is $CH_3$;
$R_2$ and $R_3$ are $CH_2CH_3$ or $R_2$ and $R_3$ are taken together as $-(CH_2)n-$ where n is 4 or 5;
$R_4$ is $CH_3$;
$R_5$ and $R_6$ are $CH_3$;
$R_7$ is $CH_3$ or $CH_2CH_3$; and
$R_8$ is $CH_2CH_3$ or $CH(CH_3)_2$.

2. The compounds of Preferred 1 wherein J is J-3.
3. The compounds of Preferred 1 wherein J is J-2.
4. The compounds of Preferred 1 wherein J is J-1.

Specifically preferred compounds of the invention for reasons of most efficient weed control and/or better crop tolerance are compounds of Formula I selected from the group consisting of:

exo-1-methyl-4-(1-methylethyl)-2-[(4′-thiazolyl)methoxy]-7-oxabicyclo[2.2.1]heptane;
exo-1,4-diethyl-2-[(4′-thiazolyl)methoxy]-7-oxabicyclo[2.2.1]heptane;
exo-1-methyl-4-(1-methylethyl)-2-[(2′-(6′-fluoropyridyl))methoxy]-7-oxabicyclo[2.2.1]heptane; and
exo-1,4-diethyl-2-[(2′-(6′-fluoropyridyl))methoxy]-7-oxabicyclo[2.2.1]heptane.

## Detailed Description of the Invention

Many of the compounds of Formula I are known in the art. They can be prepared according to processes described in U.S. 4,670,041 and U.S. 4,486,219.

As an exemplification, compounds of Formula I wherein Q is Q-1 and J is J-1 can be prepared as described in Scheme 1.

Scheme 1

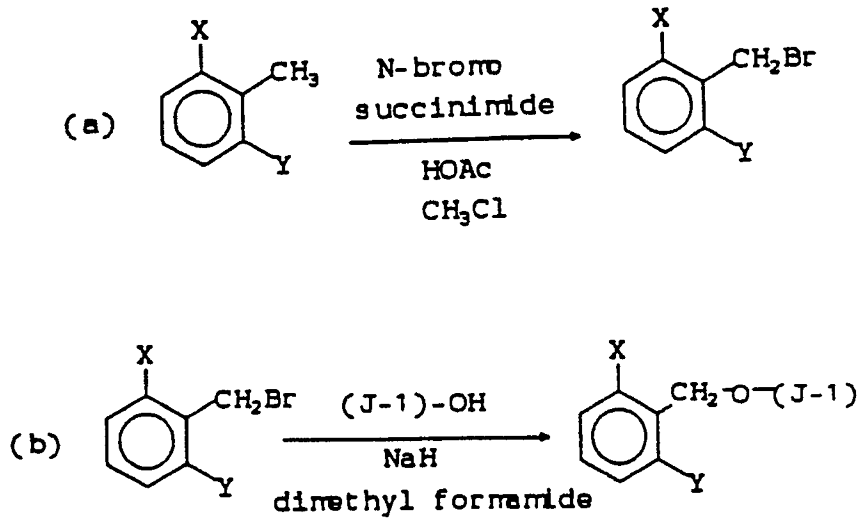


The precursor substituted toluenes can be brominated at the benzylic position as shown in Scheme 1a, and side products can be removed by purification methods (such as crystallization or chromatography) well known in the art. The substituted benzylic bromide can be coupled to the J-1 alcohol by a Williamson Ether Synthesis, effected by sodium hydride as in Scheme 1b.

Compounds of Formula I wherein Q is Q-2, Q-3 or Q-4 are prepared by a related process shown in Scheme 2 in the case of Q-2 with $R_9$ = H and $R_{10}$ = F in the 6-position.

Scheme 2

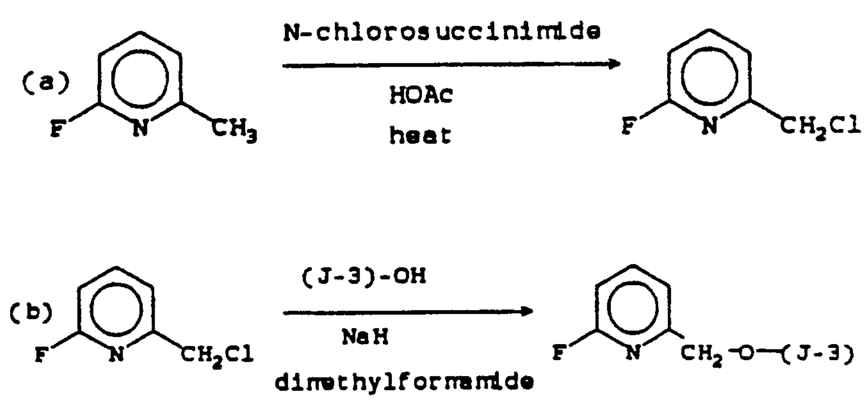

An alternate synthesis of compounds of this type is shown in Scheme 3. This procedure involves metallation of a nicotinate ester and chlorination,

## Scheme 3

(a)

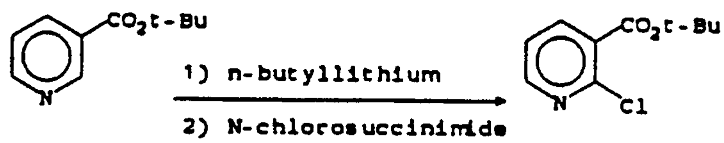


(b)

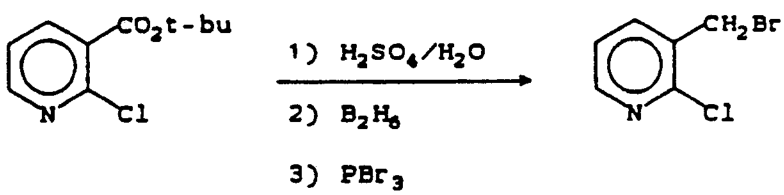


(c)

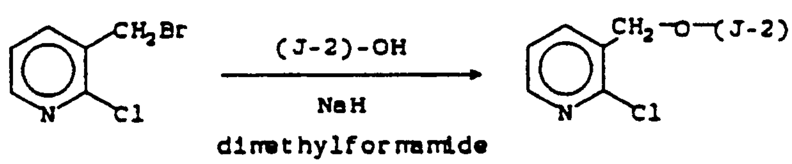


followed by acid hydrolysis, borane reduction and bromination of the resulting alcohol to furnish the substituted nicotinyl bromide, which may be coupled with a J-2 alcohol by the Williamson Ether Synthesis.

Another variant can be employed in the case of compounds of Formula I wherein Q = Q-14. This uses a Hantzsch-type synthesis as shown in Scheme 4.

Scheme 4

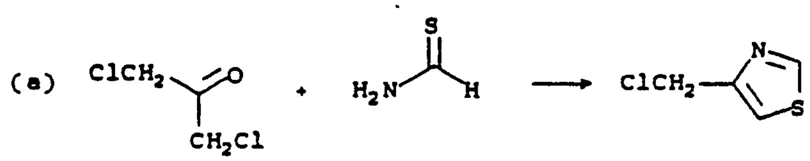

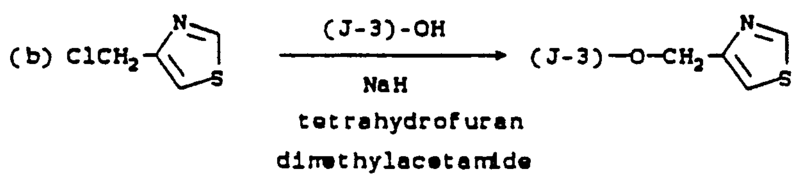


The methodology described in Schemes 1-4 is also applicable to other various Q groups as outlined in Scheme 5.

## Scheme 5

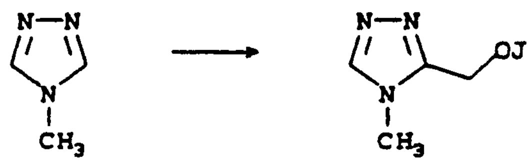

3

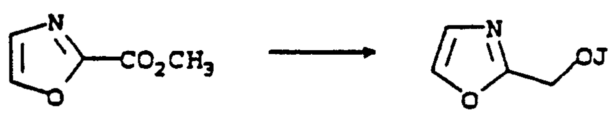

4

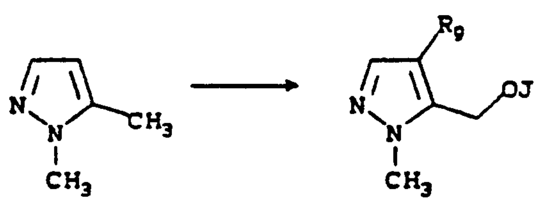

5

Appropriate heterocyclic starting materials such as 3, 4 and 5 are known in the art. Analagous functional group manipulation as taught in Schemes 1-4 and applied to intermediates 3, 4 and 5 are also known in the art.

All stereoisomers (diastereomers and enantiomers); endo and exo forms; and mixtures thereof are included within the scope of the present invention.

The various individual isomeric forms and various combinations of the derivatives usually have some difference in herbicidal or plant growth control properties.

The following examples serve to illustrate the preparation of compounds of the invention.

## Example 1

### 4-(Chloromethyl)thiazole hydrochloride

A vessel containing 44.25 mL of formamide (1.11 mol) was cooled to 0 °C and stirred vigorously during the portionwise addition (over 1 1/2 hour) of 25.000 g of phosphorus pentasulfide (0.056 mol). When the addition was complete, 250 mL of ether was added and the suspension was stirred at room temperature overnight. The suspension was filtered, and the solids were triturated four times with 100 mL of ether. The combined ether portions were concentrated to yield 18.89 g of crude thioformamide as an orange liquid (35% yield).

This portion of crude thioformamide (0.385 mol) was dissolved in 315 mL of acetone and this was added to a solution of 31.95 g of 1,3-dichloroacetone (0.252 mol) in 126 mL of acetone. This mixture was stirred at room temperature for five days, and was then filtered to give 19.96 g of 4-(chloromethyl) thiazole hydrochloride as an off-white solid (50% yield). A small sample was neutralized for spectral characterization. Proton NMR Spectrum ($CDCl_3$, 90 MHz, ppm): 4.81 (s, 2H); 7.44 (s, 1H); 8.86 (s, 1H).

## Example 2

### exo-1-Methyl-4-(1-methylethyl)-2-[(4-thiazolyl)methoxyl-7-oxabicyclo[2.2.1]heptane

A stirred suspension of 7.067 g of 80% dispersion of sodium hydride in mineral oil (177 mmol) in 80 mL of dry tetrahydrofuran was charged with 6.01 g of exo-1-methyl-4-(1-methylethyl)-7-oxabicyclo[2.2.1]-heptan-2-ol (35.3 mmol). This mixture was diluted to 150 mL with additional tetrahydrofuran, and was then charged with 75 mL of dimethylacetamide followed by 5.858 g of 4-(chloromethyl)thiazole hydrochloride (37.1 mmol) and 5.268 g of sodium iodide (35.4 mmol). The reaction vessel was wrapped in aluminum foil to protect from light, and was then heated to reflux for 8 hrs, and then stirred at room temperature for 2 1/2 days. The reaction mixture was poured into a mixture of 500 mL of saturated aqueous ammonium chloride and 200 mL of water, and this was extracted four times with 60 mL of ether. The combined ether portions were extracted wtih 150 mL of brine, dried ($MgSO_4$) and concentrated to give 12.669 g of a dark brown liquid. This was purified on 250 g of silica using as eluent first 5% acetone in petroleum ether, followed by 10% and 20%, to give a major fraction of 2.962 g of exo-1-methyl-4-(1-methylethyl)-2-[(4-thiazolyl)-methoxy]-7-oxabicyclo[2.2.1]heptane as a yellow liquid (31% yield).

Proton NMR ($CDCl_3$, 90 MHz, ppm): 0.98 (d, 6H, J = 7 Hz); 1.49 (s, 3H); 1.3-1.7 (m, 4H); 1.8-2.2 (m, 3H); 3.68 (dd, 1H, J = 2.8, 6.5 Hz); 4.57 (d, 1H, J = 12 Hz); 4.78 (d, 1H, J = 12 Hz); 7.31 (fine m, 1H); 8.75 (d, 1H, J = 1.5 Hz).

Carbon NMR ($CDCl_3$, 100 MHz, ppm): 16.4, 17.9, 18.1, 31.6, 32.5, 33.7, 42.2, 66.6, 83.7, 85.2, 88.4, 114.9, 152.5, 155.3.

## Example 3

### exo-1,4-diethyl-2-[(4-thiazolyl)methoxy]-7-oxabicyclo[2.2.1]heptane

A stirred suspension of 361 mg of an 80% suspension of sodium hydride in mineral oil (12.0 mmol) in 4 mL of dry tetrahydrofuran was charged with a solution of 505 mg of exo-1,4-diethyl-7-oxabicyclo[2.2.1]-heptan-2-ol (2.97 mmol) in a total of 6 mL of tetrahydrofuran. This mixture was charged with 5 mL of dimethylacetamide, followed by 851 mg of 4-(chloromethyl)thiazole hydrochloride (5.38 mmol) and 445 mg of sodium iodide (2.99 mmol). The resulting mixture was heated to reflux under nitrogen for six hrs. The reaction mixture was poured into 100 mL of saturated aqueous ammonium chloride, and this was extracted

three times with 20 mL of ether. The combined ether layers were extracted with 50 mL of brine, dried ($MgSO_4$) and concentrated to give 969 mg of an orange oil. This was purified by chromatography on 30 g of silica, using as eluent 5% acetone in petroleum ether, followed by 10% and 15%, to yield 158 mg of exo-1,4-diethyl-2-[(4-thiazolyl)methoxy]-7-oxabicyclo[2.2.1]heptane as a pale yellow oil (21% yield). Proton NMR ($CDCl_3$, 90 MHz, ppm): 0.93 (t, 3H, J = 7.5 Hz); 0.98 (t, 3H, J = 7.5 Hz); 1.3-2.3 (m, 10H); 3.75 (dd, 1H, J = 2.5, 7 Hz); 4.52 (d, 1H, J = 12 Hz); 4.73 (d, 1H, J = 12 Hz); 7.29 (fine m, 1H); 8.77 (d, 1H, J = 2.5 Hz).

Using the procedures described above, the compounds of Tables I-IV may be prepared.

TABLE I

| $JOCH_2Q$ | | | | |
|---|---|---|---|---|
| J is J-1 | | | | |
| Q is Q-1 | | | | |
| $R_1$ | $R_2$ | $R_3$ | X | Y |
| $CH_3$ | $CH_2CH_3$ | $CH_2CH_3$ | H | 2-Cl |
| $CH_3$ | $CH_2CH_3$ | $CH_2CH_3$ | 2-F | 6-Cl |
| $CH_3$ | $CH_2CH_3$ | $CH_2CH_3$ | 2-F | 6-F |
| $CH_3$ | $CH_2CH_3$ | $CH_2CH_3$ | $2-CH_3$ | H |

TABLE II

| $JOCH_2Q$ | | | | |
|---|---|---|---|---|
| J is J-1 | | | | |
| Q is Q-2 | | | | |
| $R_9$ is in the 3 position | | | | |
| $R_1$ | $R_2$ | $R_3$ | $R_9$ | $R_{10}$ |
| $CH_3$ | $CH_3$ | $CH_3$ | F | H |
| $CH_3$ | $CH_3$ | $CH_2CH_3$ | F | H |
| $CH_3$ | $CH_2CH_3$ | $CH_2CH_3$ | F | H |
| $CH_3$ | $CH_2CH_3$ | $CH_2CH_3$ | Cl | H |
| $CH_3$ | $CH_2CH_3$ | $CH_2CH_3$ | Br | H |
| $CH_3$ | $CH_2CH_3$ | $CH_2CH_3$ | $CH_3$ | H |
| $CH_3$ | $CH_2CH_3$ | $CH_2CH_3$ | $CH_2CH_3$ | H |
| $CH_3$ | $CH_2CH_3$ | $CH_2CH_3$ | Br | 4-F |
| $CH_3$ | cyclopentyl | | F | H |
| $CH_3$ | cyclopentyl | | Cl | H |
| $CH_3$ | cyclopentyl | | Br | H |
| $CH_3$ | cyclopentyl | | $CH_3$ | H |
| $CH_3$ | cyclopentyl | | $CH_2CH_3$ | H |
| $CH_3$ | cyclohexyl | | F | H |
| $CH_3$ | cyclohexyl | | Cl | H |
| $CH_3$ | cyclohexyl | | Br | H |
| $CH_3$ | cyclohexyl | | $CH_3$ | H |
| $CH_2CH_3$ | $CH_3$ | $CH_3$ | F | H |
| $CH_3$ | $CH_3$ | $CH_3$ | H | 6-F |
| $CH_3$ | $CH_2CH_3$ | $CH_2CH_3$ | H | 6-F |
| $CH_3$ | cyclopentyl | | H | 6-F |
| $CH_3$ | cyclohexyl | | H | 6-F |

TABLE IIa

| JOC$H_2$Q | | | | |
|---|---|---|---|---|
| J is J-1 | | | | |
| Q is Q-3 | | | | |
| $R_9$ is in the 2 position | | | | |
| $R_1$ | $R_2$ | $R_3$ | $R_9$ | $R_{10}$ |
| $CH_3$ | $CH_3$ | $CH_3$ | F | H |
| $CH_3$ | $CH_3$ | $CH_2CH_3$ | F | H |
| $CH_3$ | $CH_2CH_3$ | $CH_2CH_3$ | F | H |
| $CH_3$ | $CH_2CH_3$ | $CH_2CH_3$ | Cl | H |
| $CH_3$ | $CH_2CH_3$ | $CH_2CH_3$ | Br | H |
| $CH_3$ | $CH_2CH_3$ | $CH_2CH_3$ | $CH_3$ | H |
| $CH_3$ | $CH_2CH_3$ | $CH_2CH_3$ | $CH_2CH_3$ | H |
| $CH_3$ | $CH_2CH_3$ | $CH_2CH_3$ | Br | 4-F |
| $CH_3$ | cyclopentyl | | F | H |
| $CH_3$ | cyclopentyl | | Cl | H |
| $CH_3$ | cyclopentyl | | Br | H |
| $CH_3$ | cyclopentyl | | $CH_3$ | H |
| $CH_3$ | cyclopentyl | | $CH_2CH_3$ | H |
| $CH_3$ | cyclohexyl | | F | H |
| $CH_3$ | cyclohexyl | | Cl | H |
| $CH_3$ | cyclohexyl | | Br | H |
| $CH_3$ | cyclohexyl | | $CH_3$ | H |
| $CH_2CH_3$ | $CH_3$ | $CH_3$ | F | H |
| $CH_3$ | $CH_3$ | $CH_3$ | F | 4-F |
| $CH_3$ | $CH_2CH_3$ | $CH_2CH_3$ | F | 4-Cl |
| $CH_3$ | cyclopentyl | | F | 4-F |
| $CH_3$ | cyclohexyl | | F | 4-Cl |
| $CH_3$ | $CH_3$ | $CH_3$ | H | H |
| $CH_3$ | $CH_2CH_3$ | $CH_2CH_3$ | H | H |
| $CH_3$ | cyclopentyl | | H | H |
| $CH_3$ | cyclohexyl | | H | H |

TABLE IIb

| JOCH$_2$Q | | | | |
|---|---|---|---|---|
| J is J-1 | | | | |
| Q is Q-4 | | | | |
| R$_9$ is in the 3 position | | | | |
| R$_1$ | R$_2$ | R$_3$ | R$_9$ | R$_{10}$ |
| CH$_3$ | CH$_3$ | CH$_3$ | F | H |
| CH$_3$ | CH$_3$ | CH$_2$CH$_3$ | F | H |
| CH$_3$ | CH$_2$CH$_3$ | CH$_2$CH$_3$ | F | H |
| CH$_3$ | CH$_2$CH$_3$ | CH$_2$CH$_3$ | Cl | H |
| CH$_3$ | CH$_2$CH$_3$ | CH$_2$CH$_3$ | Br | H |
| CH$_3$ | CH$_2$CH$_3$ | CH$_2$CH$_3$ | CH$_3$ | H |
| CH$_3$ | CH$_2$CH$_3$ | CH$_2$CH$_3$ | CH$_2$CH$_3$ | H |
| CH$_3$ | CH$_2$CH$_3$ | CH$_2$CH$_3$ | Br | 5-F |
| CH$_3$ | cyclopentyl | | F | H |
| CH$_3$ | cyclopentyl | | Cl | H |
| CH$_3$ | cyclopentyl | | Br | H |
| CH$_3$ | cyclopentyl | | CH$_3$ | H |
| CH$_3$ | cyclopentyl | | CH$_2$CH$_3$ | H |
| CH$_3$ | cyclohexyl | | F | H |
| CH$_3$ | cyclohexyl | | Cl | H |
| CH$_3$ | cyclohexyl | | Br | H |
| CH$_3$ | cyclohexyl | | CH$_3$ | H |
| CH$_2$CH$_3$ | CH$_3$ | CH$_3$ | F | H |
| CH$_3$ | CH$_3$ | CH$_3$ | H | H |
| CH$_3$ | CH$_2$CH$_3$ | CH$_2$CH$_3$ | H | H |
| CH$_3$ | cyclopentyl | | H | H |
| CH$_3$ | cyclohexyl | | H | H |

TABLE IIc

| JOCH$_2$Q | | | | |
|---|---|---|---|---|
| J is J-1 | | | | |
| Q is Q-5 | | | | |
| $R_9$ is in the 4 position | | | | |
| $R_1$ | $R_2$ | $R_3$ | $R_9$ | $R_{10}$ |
| $CH_3$ | $CH_3$ | $CH_3$ | F | H |
| $CH_3$ | $CH_3$ | $CH_2CH_3$ | F | H |
| $CH_3$ | $CH_2CH_3$ | $CH_2CH_3$ | F | H |
| $CH_3$ | $CH_2CH_3$ | $CH_2CH_3$ | Cl | H |
| $CH_3$ | $CH_2CH_3$ | $CH_2CH_3$ | Br | H |
| $CH_3$ | $CH_2CH_3$ | $CH_2CH_3$ | $CH_3$ | H |
| $CH_3$ | $CH_2CH_3$ | $CH_2CH_3$ | $CH_2CH_3$ | H |
| $CH_3$ | $CH_2CH_3$ | $CH_2CH_3$ | Br | 5-F |
| $CH_3$ | cyclopentyl | | F | H |
| $CH_3$ | cyclopentyl | | Cl | H |
| $CH_3$ | cyclopentyl | | Br | H |
| $CH_3$ | cyclopentyl | | $CH_3$ | H |
| $CH_3$ | cyclopentyl | | $CH_2CH_3$ | H |
| $CH_3$ | cyclohexyl | | F | H |
| $CH_3$ | cyclohexyl | | Cl | H |
| $CH_3$ | cyclohexyl | | Br | H |
| $CH_3$ | cyclohexyl | | $CH_3$ | H |
| $CH_2CH_3$ | $CH_3$ | $CH_3$ | F | H |
| $CH_3$ | $CH_3$ | $CH_3$ | H | H |
| $CH_3$ | $CH_2CH_3$ | $CH_2CH_3$ | H | H |
| $CH_3$ | cyclopentyl | | H | H |
| $CH_3$ | cyclohexyl | | H | H |

TABLE IId

| JOCH$_2$Q | | | | |
|---|---|---|---|---|
| J is J-1 | | | | |
| Q is Q-6 | | | | |
| $R_9$ is in the 3 position | | | | |
| $R_1$ | $R_2$ | $R_3$ | $R_9$ | $R_{10}$ |
| $CH_3$ | $CH_3$ | $CH_3$ | F | H |
| $CH_3$ | $CH_3$ | $CH_2CH_3$ | F | H |
| $CH_3$ | $CH_2CH_3$ | $CH_2CH_3$ | F | H |
| $CH_3$ | $CH_2CH_3$ | $CH_2CH_3$ | Cl | H |
| $CH_3$ | $CH_2CH_3$ | $CH_2CH_3$ | Br | H |
| $CH_3$ | $CH_2CH_3$ | $CH_2CH_3$ | $CH_3$ | H |
| $CH_3$ | $CH_2CH_3$ | $CH_2CH_3$ | $CH_2CH_3$ | H |
| $CH_3$ | $CH_2CH_3$ | $CH_2CH_3$ | Br | 5-F |
| $CH_3$ | cyclopentyl | | F | H |
| $CH_3$ | cyclopentyl | | Cl | H |
| $CH_3$ | cyclopentyl | | Br | H |
| $CH_3$ | cyclopentyl | | $CH_3$ | H |
| $CH_3$ | cyclopentyl | | $CH_2CH_3$ | H |
| $CH_3$ | cyclohexyl | | F | H |
| $CH_3$ | cyclohexyl | | Cl | H |
| $CH_3$ | cyclohexyl | | Br | H |
| $CH_3$ | cyclohexyl | | $CH_3$ | H |
| $CH_2CH_3$ | $CH_3$ | $CH_3$ | F | H |
| $CH_3$ | $CH_3$ | $CH_3$ | H | H |
| $CH_3$ | $CH_2CH_3$ | $CH_2CH_3$ | H | H |
| $CH_3$ | cyclopentyl | | H | H |
| $CH_3$ | cyclohexyl | | H | H |

TABLE IIe

| JOCH₂Q | | | | |
|---|---|---|---|---|
| J is J-1 | | | | |
| Q is Q-7 | | | | |
| $R_9$ is in the 4 position | | | | |
| $R_1$ | $R_2$ | $R_3$ | $R_9$ | $R_{10}$ |
| $CH_3$ | $CH_3$ | $CH_3$ | F | H |
| $CH_3$ | $CH_3$ | $CH_2CH_3$ | F | H |
| $CH_3$ | $CH_2CH_3$ | $CH_2CH_3$ | F | H |
| $CH_3$ | $CH_2CH_3$ | $CH_2CH_3$ | Cl | H |
| $CH_3$ | $CH_2CH_3$ | $CH_2CH_3$ | Br | H |
| $CH_3$ | $CH_2CH_3$ | $CH_2CH_3$ | $CH_3$ | H |
| $CH_3$ | $CH_2CH_3$ | $CH_2CH_3$ | $CH_2CH_3$ | H |
| $CH_3$ | $CH_2CH_3$ | $CH_2CH_3$ | Br | 5-F |
| $CH_3$ | cyclopentyl | | F | H |
| $CH_3$ | cyclopentyl | | Cl | H |
| $CH_3$ | cyclopentyl | | Br | H |
| $CH_3$ | cyclopentyl | | $CH_3$ | H |
| $CH_3$ | cyclopentyl | | $CH_2CH_3$ | H |
| $CH_3$ | cyclohexyl | | F | H |
| $CH_3$ | cyclohexyl | | Cl | H |
| $CH_3$ | cyclohexyl | | Br | H |
| $CH_3$ | cyclohexyl | | $CH_3$ | H |
| $CH_2CH_3$ | $CH_3$ | $CH_3$ | F | H |
| $CH_3$ | $CH_3$ | $CH_3$ | H | H |
| $CH_3$ | $CH_2CH_3$ | $CH_2CH_3$ | H | H |
| $CH_3$ | cyclopentyl | | H | H |
| $CH_3$ | cyclohexyl | | H | H |

TABLE IIf

| JOCH₂Q | | | |
|---|---|---|---|
| J is J-1 | | | |
| Q is Q-8 | | | |
| $R_9$ is in the 5 position | | | |
| $R_1$ | $R_2$ | $R_3$ | $R_9$ |
| $CH_3$ | $CH_3$ | $CH_3$ | F |
| $CH_3$ | $CH_3$ | $CH_2CH_3$ | F |
| $CH_3$ | $CH_2CH_3$ | $CH_2CH_3$ | F |
| $CH_3$ | $CH_2CH_3$ | $CH_2CH_3$ | Cl |
| $CH_3$ | $CH_2CH_3$ | $CH_2CH_3$ | Br |
| $CH_3$ | $CH_2CH_3$ | $CH_2CH_3$ | $CH_3$ |
| $CH_3$ | $CH_2CH_3$ | $CH_2CH_3$ | $CH_2CH_3$ |
| $CH_3$ | $CH_2CH_3$ | $CH_2CH_3$ | Br |
| $CH_3$ | cyclopentyl | | F |
| $CH_3$ | cyclopentyl | | Cl |
| $CH_3$ | cyclopentyl | | Br |
| $CH_3$ | cyclopentyl | | $CH_3$ |
| $CH_3$ | cyclopentyl | | $CH_2CH_3$ |
| $CH_3$ | cyclohexyl | | F |
| $CH_3$ | cyclohexyl | | Cl |
| $CH_3$ | cyclohexyl | | Br |
| $CH_3$ | cyclohexyl | | $CH_3$ |
| $CH_2CH_3$ | $CH_3$ | $CH_3$ | F |
| $CH_3$ | $CH_3$ | $CH_3$ | H |
| $CH_3$ | $CH_2CH_3$ | $CH_2CH_3$ | H |
| $CH_3$ | cyclopentyl | | H |
| $CH_3$ | cyclohexyl | | H |

TABLE IIg

| JOCH$_2$Q | | | | |
|---|---|---|---|---|
| J is J-1 | | | | |
| Q is Q-9 | | | | |
| $R_9$ is in the 4 position | | | | |
| $R_1$ | $R_2$ | $R_3$ | $R_9$ | $R_{10}$ |
| $CH_3$ | $CH_3$ | $CH_3$ | F | H |
| $CH_3$ | $CH_3$ | $CH_2CH_3$ | F | H |
| $CH_3$ | $CH_2CH_3$ | $CH_2CH_3$ | F | H |
| $CH_3$ | $CH_2CH_3$ | $CH_2CH_3$ | Cl | H |
| $CH_3$ | $CH_2CH_3$ | $CH_2CH_3$ | Br | H |
| $CH_3$ | $CH_2CH_3$ | $CH_2CH_3$ | $CH_3$ | H |
| $CH_3$ | $CH_2CH_3$ | $CH_2CH_3$ | $CH_2CH_3$ | H |
| $CH_3$ | $CH_2CH_3$ | $CH_2CH_3$ | Br | 5-F |
| $CH_3$ | cyclopentyl | | F | H |
| $CH_3$ | cyclopentyl | | Cl | H |
| $CH_3$ | cyclopentyl | | Br | H |
| $CH_3$ | cyclopentyl | | $CH_3$ | H |
| $CH_3$ | cyclopentyl | | $CH_2CH_3$ | H |
| $CH_3$ | cyclohexyl | | F | H |
| $CH_3$ | cyclohexyl | | Cl | H |
| $CH_3$ | cyclohexyl | | Br | H |
| $CH_3$ | cyclohexyl | | $CH_3$ | H |
| $CH_2CH_3$ | $CH_3$ | $CH_3$ | F | H |
| $CH_3$ | $CH_3$ | $CH_3$ | H | H |
| $CH_3$ | $CH_2CH_3$ | $CH_2CH_3$ | H | H |
| $CH_3$ | cyclopentyl | | H | H |
| $CH_3$ | cyclohexyl | | H | H |

TABLE IIh

| JOCH₂Q | | | | |
|---|---|---|---|---|
| J is J-1 | | | | |
| Q is Q-10 | | | | |
| $R_9$ is in the 5 position | | | | |
| $R_1$ | $R_2$ | $R_3$ | $R_9$ | $R_{10}$ |
| $CH_3$ | $CH_3$ | $CH_3$ | F | H |
| $CH_3$ | $CH_3$ | $CH_2CH_3$ | F | H |
| $CH_3$ | $CH_2CH_3$ | $CH_2CH_3$ | F | H |
| $CH_3$ | $CH_2CH_3$ | $CH_2CH_3$ | Cl | H |
| $CH_3$ | $CH_2CH_3$ | $CH_2CH_3$ | Br | H |
| $CH_3$ | $CH_2CH_3$ | $CH_2CH_3$ | $CH_3$ | H |
| $CH_3$ | $CH_2CH_3$ | $CH_2CH_3$ | $CH_2CH_3$ | H |
| $CH_3$ | $CH_2CH_3$ | $CH_2CH_3$ | Br | 2-F |
| $CH_3$ | cyclopentyl | | F | H |
| $CH_3$ | cyclopentyl | | Cl | H |
| $CH_3$ | cyclopentyl | | Br | H |
| $CH_3$ | cyclopentyl | | $CH_3$ | H |
| $CH_3$ | cyclopentyl | | $CH_2CH_3$ | H |
| $CH_3$ | cyclohexyl | | F | H |
| $CH_3$ | cyclohexyl | | Cl | H |
| $CH_3$ | cyclohexyl | | Br | H |
| $CH_3$ | cyclohexyl | | $CH_3$ | H |
| $CH_2CH_3$ | $CH_3$ | $CH_3$ | F | H |
| $CH_3$ | $CH_3$ | $CH_3$ | H | H |
| $CH_3$ | $CH_2CH_3$ | $CH_2CH_3$ | H | H |
| $CH_3$ | cyclopentyl | | H | H |
| $CH_3$ | cyclohexyl | | H | H |

TABLE IIi

| JOCH$_2$Q | | | | |
|---|---|---|---|---|
| J is J-1 | | | | |
| Q is Q-11 | | | | |
| $R_9$ is in the 4 position | | | | |
| $R_1$ | $R_2$ | $R_3$ | $R_9$ | $R_{10}$ |
| $CH_3$ | $CH_3$ | $CH_3$ | F | H |
| $CH_3$ | $CH_3$ | $CH_2CH_3$ | F | H |
| $CH_3$ | $CH_2CH_3$ | $CH_2CH_3$ | F | H |
| $CH_3$ | $CH_2CH_3$ | $CH_2CH_3$ | Cl | H |
| $CH_3$ | $CH_2CH_3$ | $CH_2CH_3$ | Br | H |
| $CH_3$ | $CH_2CH_3$ | $CH_2CH_3$ | $CH_3$ | H |
| $CH_3$ | $CH_2CH_3$ | $CH_2CH_3$ | $CH_2CH_3$ | H |
| $CH_3$ | $CH_2CH_3$ | $CH_2CH_3$ | Br | 3-F |
| $CH_3$ | cyclopentyl | | F | H |
| $CH_3$ | cyclopentyl | | Cl | H |
| $CH_3$ | cyclopentyl | | Br | H |
| $CH_3$ | cyclopentyl | | $CH_3$ | H |
| $CH_3$ | cyclopentyl | | $CH_2CH_3$ | H |
| $CH_3$ | cyclohexyl | | F | H |
| $CH_3$ | cyclohexyl | | Cl | H |
| $CH_3$ | cyclohexyl | | Br | H |
| $CH_3$ | cyclohexyl | | $CH_3$ | H |
| $CH_2CH_3$ | $CH_3$ | $CH_3$ | F | H |
| $CH_3$ | $CH_3$ | $CH_3$ | H | H |
| $CH_3$ | $CH_2CH_3$ | $CH_2CH_3$ | H | H |
| $CH_3$ | cyclopentyl | | H | H |
| $CH_3$ | cyclohexyl | | H | H |

TABLE IIj

| JOCH$_2$Q | | | | |
|---|---|---|---|---|
| J is J-1 | | | | |
| Q is Q-12 | | | | |
| R$_9$ is in the 4 position | | | | |
| R$_1$ | R$_2$ | R$_3$ | R$_9$ | R$_{10}$ |
| CH$_3$ | CH$_3$ | CH$_3$ | F | H |
| CH$_3$ | CH$_3$ | CH$_2$CH$_3$ | F | H |
| CH$_3$ | CH$_2$CH$_3$ | CH$_2$CH$_3$ | F | H |
| CH$_3$ | CH$_2$CH$_3$ | CH$_2$CH$_3$ | Cl | H |
| CH$_3$ | CH$_2$CH$_3$ | CH$_2$CH$_3$ | Br | H |
| CH$_3$ | CH$_2$CH$_3$ | CH$_2$CH$_3$ | CH$_3$ | H |
| CH$_3$ | CH$_2$CH$_3$ | CH$_2$CH$_3$ | CH$_2$CH$_3$ | H |
| CH$_3$ | CH$_2$CH$_3$ | CH$_2$CH$_3$ | Br | 5-F |
| CH$_3$ | cyclopentyl | | F | H |
| CH$_3$ | cyclopentyl | | Cl | H |
| CH$_3$ | cyclopentyl | | Br | H |
| CH$_3$ | cyclopentyl | | CH$_3$ | H |
| CH$_3$ | cyclopentyl | | CH$_2$CH$_3$ | H |
| CH$_3$ | cyclohexyl | | F | H |
| CH$_3$ | cyclohexyl | | Cl | H |
| CH$_3$ | cyclohexyl | | Br | H |
| CH$_3$ | cyclohexyl | | CH$_3$ | H |
| CH$_2$CH$_3$ | CH$_3$ | CH$_3$ | F | H |
| CH$_3$ | CH$_3$ | CH$_3$ | H | H |
| CH$_3$ | CH$_2$CH$_3$ | CH$_2$CH$_3$ | H | H |
| CH$_3$ | cyclopentyl | | H | H |
| CH$_3$ | cyclohexyl | | H | H |

TABLE IIk

| JOCH₂Q | | | | |
|---|---|---|---|---|
| J is J-1 | | | | |
| Q is Q-13 | | | | |
| $R_9$ is in the 4 position | | | | |
| $R_1$ | $R_2$ | $R_3$ | $R_9$ | $R_{10}$ |
| $CH_3$ | $CH_3$ | $CH_3$ | F | H |
| $CH_3$ | $CH_3$ | $CH_2CH_3$ | F | H |
| $CH_3$ | $CH_2CH_3$ | $CH_2CH_3$ | F | H |
| $CH_3$ | $CH_2CH_3$ | $CH_2CH_3$ | Cl | H |
| $CH_3$ | $CH_2CH_3$ | $CH_2CH_3$ | Br | H |
| $CH_3$ | $CH_2CH_3$ | $CH_2CH_3$ | $CH_3$ | H |
| $CH_3$ | $CH_2CH_3$ | $CH_2CH_3$ | $CH_2CH_3$ | H |
| $CH_3$ | $CH_2CH_3$ | $CH_2CH_3$ | Br | 2-F |
| $CH_3$ | cyclopentyl | | F | H |
| $CH_3$ | cyclopentyl | | Cl | H |
| $CH_3$ | cyclopentyl | | Br | H |
| $CH_3$ | cyclopentyl | | $CH_3$ | H |
| $CH_3$ | cyclopentyl | | $CH_2CH_3$ | H |
| $CH_3$ | cyclohexyl | | F | H |
| $CH_3$ | cyclohexyl | | Cl | H |
| $CH_3$ | cyclohexyl | | Br | H |
| $CH_3$ | cyclohexyl | | $CH_3$ | H |
| $CH_2CH_3$ | $CH_3$ | $CH_3$ | F | H |
| $CH_3$ | $CH_3$ | $CH_3$ | H | H |
| $CH_3$ | $CH_2CH_3$ | $CH_2CH_3$ | H | H |
| $CH_3$ | cyclopentyl | | H | H |
| $CH_3$ | cyclohexyl | | H | H |

TABLE III

| JOCH$_2$Q | | | | |
|---|---|---|---|---|
| J is J-1 | | | | |
| Q is Q-14 | | | | |
| $R_9$ is in the 5 position | | | | |
| $R_1$ | $R_2$ | $R_3$ | $R_9$ | $R_{10}$ |
| $CH_3$ | $CH_3$ | $CH_3$ | F | H |
| $CH_3$ | $CH_3$ | $CH_2CH_3$ | F | H |
| $CH_3$ | $CH_2CH_3$ | $CH_2CH_3$ | F | H |
| $CH_3$ | $CH_2CH_3$ | $CH_2CH_3$ | Cl | H |
| $CH_3$ | $CH_2CH_3$ | $CH_2CH_3$ | Br | H |
| $CH_3$ | $CH_2CH_3$ | $CH_2CH_3$ | $CH_3$ | H |
| $CH_3$ | $CH_2CH_3$ | $CH_2CH_3$ | $CH_2CH_3$ | H |
| $CH_3$ | $CH_2CH_3$ | $CH_2CH_3$ | Br | 2-F |
| $CH_3$ | cyclopentyl | | F | H |
| $CH_3$ | cyclopentyl | | Cl | H |
| $CH_3$ | cyclopentyl | | Br | H |
| $CH_3$ | cyclopentyl | | $CH_3$ | H |
| $CH_3$ | cyclopentyl | | $CH_2CH_3$ | H |
| $CH_3$ | cyclohexyl | | F | H |
| $CH_3$ | cyclohexyl | | Cl | H |
| $CH_3$ | cyclohexyl | | Br | H |
| $CH_3$ | cyclohexyl | | $CH_3$ | H |
| $CH_2CH_3$ | $CH_3$ | $CH_3$ | F | H |
| $CH_3$ | $CH_3$ | $CH_3$ | H | H |
| $CH_3$ | $CH_2CH_3$ | $CH_2CH_3$ | H | H |
| $CH_3$ | cyclopentyl | | H | H |
| $CH_3$ | cyclohexyl | | H | H |

TABLE III

| JOCH$_2$Q | | | | |
|---|---|---|---|---|
| J is J-2 | | | | |
| Q is Q-2 | | | | |
| R$_9$ is in the 3 position | | | | |
| $R_4$ | $R_5$ | $R_6$ | $R_9$ | $R_{10}$ |
| $CH_3$ | $CH_3$ | $CH_3$ | F | H |
| $CH_3$ | $CH_3$ | $CH_3$ | Cl | H |
| $CH_3$ | $CH_3$ | $CH_3$ | Br | H |
| $CH_3$ | $CH_3$ | $CH_3$ | $CH_3$ | H |
| $CH_3$ | $CH_3$ | $CH_3$ | $CH_2CH_3$ | H |
| $CH_3$ | $CH_3$ | $CH_2CH_3$ | Br | H |
| $CH_2CH_3$ | $CH_3$ | $CH_3$ | Br | H |
| $CH_3$ | $CH_2CH_3$ | $CH_2CH_3$ | Br | 6-F |
| $CH_3$ | $CH_3$ | $CH_3$ | H | 6-F |
| $CH_3$ | $CH_2CH_3$ | $CH_2CH_3$ | H | 6-F |
| $CH_3$ | $CH_3$ | $CH_3$ | H | H |
| $CH_3$ | $CH_2CH_3$ | $CH_2CH_3$ | H | H |

TABLE IIIa

| JOCH$_2$Q | | | | |
|---|---|---|---|---|
| J is J-2 | | | | |
| Q is Q-3 | | | | |
| R$_9$ is in the 2 position | | | | |
| $R_4$ | $R_5$ | $R_6$ | $R_9$ | $R_{10}$ |
| $CH_3$ | $CH_3$ | $CH_3$ | F | H |
| $CH_3$ | $CH_3$ | $CH_3$ | Cl | H |
| $CH_3$ | $CH_3$ | $CH_3$ | Br | H |
| $CH_3$ | $CH_3$ | $CH_3$ | $CH_3$ | H |
| $CH_3$ | $CH_3$ | $CH_3$ | $CH_2CH_3$ | H |
| $CH_3$ | $CH_3$ | $CH_2CH_3$ | Br | H |
| $CH_2CH_3$ | $CH_3$ | $CH_3$ | Br | H |
| $CH_3$ | $CH_2CH_3$ | $CH_2CH_3$ | Br | 4-F |
| $CH_3$ | $CH_3$ | $CH_3$ | F | 4-F |
| $CH_3$ | $CH_2CH_3$ | $CH_2CH_3$ | H | H |
| $CH_3$ | $CH_3$ | $CH_3$ | H | H |
| $CH_3$ | $CH_2CH_3$ | $CH_2CH_3$ | F | H |

TABLE IIIb

| JOCH$_2$Q | | | | |
|---|---|---|---|---|
| J is J-2 | | | | |
| Q is Q-4 | | | | |
| R$_9$ is in the 3 position | | | | |
| R$_4$ | R$_5$ | R$_6$ | R$_9$ | R$_{10}$ |
| CH$_3$ | CH$_3$ | CH$_3$ | F | H |
| CH$_3$ | CH$_3$ | CH$_3$ | Cl | H |
| CH$_3$ | CH$_3$ | CH$_3$ | Br | H |
| CH$_3$ | CH$_3$ | CH$_3$ | CH$_3$ | H |
| CH$_3$ | CH$_3$ | CH$_3$ | CH$_2$CH$_3$ | H |
| CH$_3$ | CH$_3$ | CH$_2$CH$_3$ | Br | H |
| CH$_2$CH$_3$ | CH$_3$ | CH$_3$ | Br | H |
| CH$_3$ | CH$_2$CH$_3$ | CH$_2$CH$_3$ | Br | 5-F |
| CH$_3$ | CH$_3$ | CH$_3$ | F | 5-F |
| CH$_3$ | CH$_2$CH$_3$ | CH$_2$CH$_3$ | F | 5-F |
| CH$_3$ | CH$_3$ | CH$_3$ | H | H |
| CH$_3$ | CH$_2$CH$_3$ | CH$_2$CH$_3$ | H | H |
| CH$_3$ | CH$_2$CH$_3$ | CH$_2$CH$_3$ | F | H |

TABLE IIIc

| JOCH$_2$Q | | | | |
|---|---|---|---|---|
| J is J-2 | | | | |
| Q is Q-5 | | | | |
| R$_9$ is in the 4 position | | | | |
| R$_4$ | R$_5$ | R$_6$ | R$_9$ | R$_{10}$ |
| CH$_3$ | CH$_3$ | CH$_3$ | F | H |
| CH$_3$ | CH$_3$ | CH$_3$ | Cl | H |
| CH$_3$ | CH$_3$ | CH$_3$ | Br | H |
| CH$_3$ | CH$_3$ | CH$_3$ | CH$_3$ | H |
| CH$_3$ | CH$_3$ | CH$_3$ | CH$_2$CH$_3$ | H |
| CH$_3$ | CH$_3$ | CH$_2$CH$_3$ | Br | H |
| CH$_2$CH$_3$ | CH$_3$ | CH$_3$ | Br | H |
| CH$_3$ | CH$_2$CH$_3$ | CH$_2$CH$_3$ | Br | 5-F |
| CH$_3$ | CH$_3$ | CH$_3$ | H | H |
| CH$_3$ | CH$_2$CH$_3$ | CH$_2$CH$_3$ | H | H |
| CH$_3$ | CH$_2$CH$_3$ | CH$_2$CH$_3$ | F | H |

TABLE IIId

| JOCH$_2$Q | | | | |
|---|---|---|---|---|
| J is J-2 | | | | |
| Q is Q-6 | | | | |
| $R_9$ is in the 3 position | | | | |
| $R_4$ | $R_5$ | $R_6$ | $R_9$ | $R_{10}$ |
| $CH_3$ | $CH_3$ | $CH_3$ | F | H |
| $CH_3$ | $CH_3$ | $CH_3$ | Cl | H |
| $CH_3$ | $CH_3$ | $CH_3$ | Br | H |
| $CH_3$ | $CH_3$ | $CH_3$ | $CH_3$ | H |
| $CH_3$ | $CH_3$ | $CH_3$ | $CH_2CH_3$ | H |
| $CH_3$ | $CH_3$ | $CH_2CH_3$ | Br | H |
| $CH_2CH_3$ | $CH_3$ | $CH_3$ | Br | H |
| $CH_3$ | $CH_2CH_3$ | $CH_2CH_3$ | Br | 5-F |
| $CH_3$ | $CH_3$ | $CH_3$ | H | H |
| $CH_3$ | $CH_2CH_3$ | $CH_2CH_3$ | H | H |
| $CH_3$ | $CH_2CH_3$ | $CH_2CH_3$ | F | H |

TABLE IIIe

| JOCH$_2$Q | | | | |
|---|---|---|---|---|
| J is J-2 | | | | |
| Q is Q-7 | | | | |
| $R_9$ is in the 4 position | | | | |
| $R_4$ | $R_5$ | $R_6$ | $R_9$ | $R_{10}$ |
| $CH_3$ | $CH_3$ | $CH_3$ | F | H |
| $CH_3$ | $CH_3$ | $CH_3$ | Cl | H |
| $CH_3$ | $CH_3$ | $CH_3$ | Br | H |
| $CH_3$ | $CH_3$ | $CH_3$ | $CH_3$ | H |
| $CH_3$ | $CH_3$ | $CH_3$ | $CH_2CH_3$ | H |
| $CH_3$ | $CH_3$ | $CH_2CH_3$ | Br | H |
| $CH_2CH_3$ | $CH_3$ | $CH_3$ | Br | H |
| $CH_3$ | $CH_2CH_3$ | $CH_2CH_3$ | Br | 5-F |
| $CH_3$ | $CH_3$ | $CH_3$ | H | H |
| $CH_3$ | $CH_2CH_3$ | $CH_2CH_3$ | H | H |
| $CH_3$ | $CH_2CH_3$ | $CH_2CH_3$ | F | H |

TABLE IIIf

| JOCH$_2$Q | | | |
|---|---|---|---|
| J is J-2 | | | |
| Q is Q-8 | | | |
| R$_9$ is in the 5 position | | | |
| R$_4$ | R$_5$ | R$_6$ | R$_9$ |
| $CH_3$ | $CH_3$ | $CH_3$ | F |
| $CH_3$ | $CH_3$ | $CH_3$ | Cl |
| $CH_3$ | $CH_3$ | $CH_3$ | Br |
| $CH_3$ | $CH_3$ | $CH_3$ | $CH_3$ |
| $CH_3$ | $CH_3$ | $CH_3$ | $CH_2CH_3$ |
| $CH_3$ | $CH_3$ | $CH_2CH_3$ | Br |
| $CH_2CH_3$ | $CH_3$ | $CH_3$ | Br |
| $CH_3$ | $CH_2CH_3$ | $CH_2CH_3$ | Br |
| $CH_3$ | $CH_3$ | $CH_3$ | H |
| $CH_3$ | $CH_2CH_3$ | $CH_2CH_3$ | H |
| $CH_3$ | $CH_2CH_3$ | $CH_2CH_3$ | F |

TABLE IIIg

| JOCH$_2$Q | | | | |
|---|---|---|---|---|
| J is J-2 | | | | |
| Q is Q-9 | | | | |
| R$_9$ is in the 4 position | | | | |
| R$_4$ | R$_5$ | R$_6$ | R$_9$ | R$_{10}$ |
| $CH_3$ | $CH_3$ | $CH_3$ | F | H |
| $CH_3$ | $CH_3$ | $CH_3$ | Cl | H |
| $CH_3$ | $CH_3$ | $CH_3$ | Br | H |
| $CH_3$ | $CH_3$ | $CH_3$ | $CH_3$ | H |
| $CH_3$ | $CH_3$ | $CH_3$ | $CH_2CH_3$ | H |
| $CH_3$ | $CH_3$ | $CH_2CH_3$ | Br | H |
| $CH_2CH_3$ | $CH_3$ | $CH_3$ | Br | H |
| $CH_3$ | $CH_2CH_3$ | $CH_2CH_3$ | Br | 5-F |
| $CH_3$ | $CH_3$ | $CH_3$ | H | H |
| $CH_3$ | $CH_2CH_3$ | $CH_2CH_3$ | H | H |
| $CH_3$ | $CH_2CH_3$ | $CH_2CH_3$ | F | H |

TABLE IIIh

| JOCH$_2$Q | | | | |
|---|---|---|---|---|
| J is J-2 | | | | |
| Q is Q-10 | | | | |
| R$_9$ is in the 5 position | | | | |
| $R_4$ | $R_5$ | $R_6$ | $R_9$ | $R_{10}$ |
| $CH_3$ | $CH_3$ | $CH_3$ | F | H |
| $CH_3$ | $CH_3$ | $CH_3$ | Cl | H |
| $CH_3$ | $CH_3$ | $CH_3$ | Br | H |
| $CH_3$ | $CH_3$ | $CH_3$ | $CH_3$ | H |
| $CH_3$ | $CH_3$ | $CH_3$ | $CH_2CH_3$ | H |
| $CH_3$ | $CH_3$ | $CH_2CH_3$ | Br | H |
| $CH_2CH_3$ | $CH_3$ | $CH_3$ | Br | H |
| $CH_3$ | $CH_2CH_3$ | $CH_2CH_3$ | Br | 3-F |
| $CH_3$ | $CH_3$ | $CH_3$ | H | H |
| $CH_3$ | $CH_2CH_3$ | $CH_2CH_3$ | H | H |
| $CH_3$ | $CH_2CH_3$ | $CH_2CH_3$ | F | H |

TABLE IIIi

| JOCH$_2$Q | | | | |
|---|---|---|---|---|
| J is J-2 | | | | |
| Q is Q-11 | | | | |
| R$_9$ is in the 4 position | | | | |
| $R_4$ | $R_5$ | $R_6$ | $R_9$ | $R_{10}$ |
| $CH_3$ | $CH_3$ | $CH_3$ | F | H |
| $CH_3$ | $CH_3$ | $CH_3$ | Cl | H |
| $CH_3$ | $CH_3$ | $CH_3$ | Br | H |
| $CH_3$ | $CH_3$ | $CH_3$ | $CH_3$ | H |
| $CH_3$ | $CH_3$ | $CH_3$ | $CH_2CH_3$ | H |
| $CH_3$ | $CH_3$ | $CH_2CH_3$ | Br | H |
| $CH_2CH_3$ | $CH_3$ | $CH_3$ | Br | H |
| $CH_3$ | $CH_2CH_3$ | $CH_2CH_3$ | Br | 3-F |
| $CH_3$ | $CH_3$ | $CH_3$ | H | H |
| $CH_3$ | $CH_2CH_3$ | $CH_2CH_3$ | H | H |
| $CH_3$ | $CH_2CH_3$ | $CH_2CH_3$ | F | H |

TABLE IIIj

| JOCH$_2$Q | | | | |
|---|---|---|---|---|
| J is J-2 | | | | |
| Q is Q-12 | | | | |
| R$_9$ is in the 3 position | | | | |
| R$_4$ | R$_5$ | R$_6$ | R$_9$ | R$_{10}$ |
| $CH_3$ | $CH_3$ | $CH_3$ | F | H |
| $CH_3$ | $CH_3$ | $CH_3$ | Cl | H |
| $CH_3$ | $CH_3$ | $CH_3$ | Br | H |
| $CH_3$ | $CH_3$ | $CH_3$ | $CH_3$ | H |
| $CH_3$ | $CH_3$ | $CH_3$ | $CH_2CH_3$ | H |
| $CH_3$ | $CH_3$ | $CH_2CH_3$ | Br | H |
| $CH_2CH_3$ | $CH_3$ | $CH_3$ | Br | H |
| $CH_3$ | $CH_3$ | $CH_3$ | H | H |
| $CH_3$ | $CH_2CH_3$ | $CH_2CH_3$ | H | H |
| $CH_3$ | $CH_2CH_3$ | $CH_2CH_3$ | F | H |

TABLE IIIk

| JOCH$_2$Q | | | | |
|---|---|---|---|---|
| J is J-2 | | | | |
| Q is Q-13 | | | | |
| R$_9$ is in the 5 position | | | | |
| R$_4$ | R$_5$ | R$_6$ | R$_9$ | R$_{10}$ |
| $CH_3$ | $CH_3$ | $CH_3$ | F | H |
| $CH_3$ | $CH_3$ | $CH_3$ | Cl | H |
| $CH_3$ | $CH_3$ | $CH_3$ | Br | H |
| $CH_3$ | $CH_3$ | $CH_3$ | $CH_3$ | H |
| $CH_3$ | $CH_3$ | $CH_3$ | $CH_2CH_3$ | H |
| $CH_3$ | $CH_3$ | $CH_2CH_3$ | Br | H |
| $CH_2CH_3$ | $CH_3$ | $CH_3$ | Br | H |
| $CH_3$ | $CH_2CH_3$ | $CH_2CH_3$ | Br | 2-F |
| $CH_3$ | $CH_3$ | $CH_3$ | H | H |
| $CH_3$ | $CH_2CH_3$ | $CH_2CH_3$ | H | H |
| $CH_3$ | $CH_2CH_3$ | $CH_2CH_3$ | F | H |

TABLE IIII

| JOCH$_2$Q | | | | |
|---|---|---|---|---|
| J is J-2 | | | | |
| Q is Q-14 | | | | |
| R$_9$ is in the 5 position | | | | |
| R$_4$ | R$_5$ | R$_6$ | R$_9$ | R$_{10}$ |
| $CH_3$ | $CH_3$ | $CH_3$ | F | H |
| $CH_3$ | $CH_3$ | $CH_3$ | Cl | H |
| $CH_3$ | $CH_3$ | $CH_3$ | Br | H |
| $CH_3$ | $CH_3$ | $CH_3$ | $CH_3$ | H |
| $CH_3$ | $CH_3$ | $CH_3$ | $CH_2CH_3$ | H |
| $CH_3$ | $CH_3$ | $CH_2CH_3$ | Br | H |
| $CH_2CH_3$ | $CH_3$ | $CH_3$ | Br | H |
| $CH_3$ | $CH_2CH_3$ | $CH_2CH_3$ | Br | 2-F |
| $CH_3$ | $CH_3$ | $CH_3$ | H | H |
| $CH_3$ | $CH_2CH_3$ | $CH_2CH_3$ | H | H |
| $CH_3$ | $CH_2CH_3$ | $CH_2CH_3$ | F | H |

TABLE IV

| JOCH$_2$Q | | | |
|---|---|---|---|
| J is J-3 | | | |
| Q is Q-2 | | | |
| R$_9$ is in the 3 position | | | |
| R$_7$ | R$_8$ | R$_9$ | R$_{10}$ |
| $CH_3$ | $CH_3$ | Br | H |
| $CH_3$ | $CH_2CH_3$ | Br | H |
| $CH_3$ | $CH(CH_3)_2$ | Br | H |
| $CH_3$ | $CH(CH_3)_2$ | F | H |
| $CH_3$ | $CH(CH_3)_2$ | Cl | H |
| $CH_3$ | $CH(CH_3)_2$ | $CH_3$ | H |
| $CH_3$ | $CH(CH_3)_2$ | $CH_2CH_3$ | H |
| $CH_2CH_3$ | $CH_2CH_3$ | F | H |
| $CH_2CH_3$ | $CH_2CH_3$ | Cl | H |
| $CH_2CH_3$ | $CH_2CH_3$ | Br | H |
| $CH_2CH_3$ | $CH_2CH_3$ | $CH_3$ | H |
| $CH_2CH_3$ | $CH_2CH_3$ | $CH_2CH_3$ | H |
| $CH_2CH_3$ | $CH_2CH_3$ | Br | 5-F |
| $CH_2CH_3$ | $CH_2CH_3$ | Br | 4-Cl |
| $CH_2CH_3$ | $CH_2CH_3$ | Br | 5-$CH_3$ |
| $CH_2CH_3$ | $CH_2CH_3$ | H | 6-F |
| $CH_3$ | $CH(CH_3)_2$ | H | 6-F |

TABLE IVa

| $JOCH_2Q$ | | | |
|---|---|---|---|
| J is J-3 | | | |
| Q is Q-3 | | | |
| $R_9$ is in the 2 position | | | |
| $R_7$ | $R_8$ | $R_9$ | $R_{10}$ |
| $CH_3$ | $CH_3$ | Br | H |
| $CH_3$ | $CH_2CH_3$ | Br | H |
| $CH_3$ | $CH(CH_3)_2$ | Br | H |
| $CH_3$ | $CH(CH_3)_2$ | F | H |
| $CH_3$ | $CH(CH_3)_2$ | Cl | H |
| $CH_3$ | $CH(CH_3)_2$ | $CH_3$ | H |
| $CH_3$ | $CH(CH_3)_2$ | $CH_2CH_3$ | H |
| $CH_2CH_3$ | $CH_2CH_3$ | F | H |
| $CH_2CH_3$ | $CH_2CH_3$ | Cl | H |
| $CH_2CH_3$ | $CH_2CH_3$ | Br | H |
| $CH_2CH_3$ | $CH_2CH_3$ | $CH_3$ | H |
| $CH_2CH_3$ | $CH_2CH_3$ | $CH_2CH_3$ | H |
| $CH_2CH_3$ | $CH_2CH_3$ | Br | 5-F |
| $CH_2CH_3$ | $CH_2CH_3$ | Br | 4-Cl |
| $CH_2CH_3$ | $CH_2CH_3$ | Br | 5-$CH_3$ |
| $CH_2CH_3$ | $CH_2CH_3$ | H | H |
| $CH_3$ | $CH(CH_3)_2$ | H | H |
| $CH_2CH_3$ | $CH_2CH_3$ | F | 4-F |
| $CH_3$ | $CH(CH_3)_2$ | F | 4-F |

TABLE IVb

| $JOCH_2Q$ | | | |
|---|---|---|---|
| J is J-3 | | | |
| Q is Q-4 | | | |
| $R_9$ is in the 3 position | | | |
| $R_7$ | $R_8$ | $R_9$ | $R_{10}$ |
| $CH_3$ | $CH_3$ | Br | H |
| $CH_3$ | $CH_2CH_3$ | Br | H |
| $CH_3$ | $CH(CH_3)_2$ | Br | H |
| $CH_3$ | $CH(CH_3)_2$ | F | H |
| $CH_3$ | $CH(CH_3)_2$ | Cl | H |
| $CH_3$ | $CH(CH_3)_2$ | $CH_3$ | H |
| $CH_3$ | $CH(CH_3)_2$ | $CH_2CH_3$ | H |
| $CH_2CH_3$ | $CH_2CH_3$ | F | H |
| $CH_2CH_3$ | $CH_2CH_3$ | Cl | H |
| $CH_2CH_3$ | $CH_2CH_3$ | Br | H |
| $CH_2CH_3$ | $CH_2CH_3$ | $CH_3$ | H |
| $CH_2CH_3$ | $CH_2CH_3$ | $CH_2CH_3$ | H |
| $CH_2CH_3$ | $CH_2CH_3$ | Br | 5-F |
| $CH_2CH_3$ | $CH_2CH_3$ | Br | 4-Cl |
| $CH_2CH_3$ | $CH_2CH_3$ | Br | 5-$CH_3$ |
| $CH_2CH_3$ | $CH_2CH_3$ | H | H |
| $CH_3$ | $CH(CH_3)_2$ | H | H |
| $CH_2CH_3$ | $CH_2CH_3$ | F | 5-F |
| $CH_3$ | $CH(CH_3)_2$ | F | 5-F |

TABLE IVc

| $JOCH_2Q$ | | | |
|---|---|---|---|
| J is J-3 | | | |
| Q is Q-5 | | | |
| $R_9$ is in the 4 position | | | |
| $R_7$ | $R_8$ | $R_9$ | $R_{10}$ |
| $CH_3$ | $CH_3$ | Br | H |
| $CH_3$ | $CH_2CH_3$ | Br | H |
| $CH_3$ | $CH(CH_3)_2$ | Br | H |
| $CH_3$ | $CH(CH_3)_2$ | F | H |
| $CH_3$ | $CH(CH_3)_2$ | Cl | H |
| $CH_3$ | $CH(CH_3)_2$ | $CH_3$ | H |
| $CH_3$ | $CH(CH_3)_2$ | $CH_2CH_3$ | H |
| $CH_2CH_3$ | $CH_2CH_3$ | F | H |
| $CH_2CH_3$ | $CH_2CH_3$ | Cl | H |
| $CH_2CH_3$ | $CH_2CH_3$ | Br | H |
| $CH_2CH_3$ | $CH_2CH_3$ | $CH_3$ | H |
| $CH_2CH_3$ | $CH_2CH_3$ | $CH_2CH_3$ | H |
| $CH_2CH_3$ | $CH_2CH_3$ | Br | 5-F |
| $CH_2CH_3$ | $CH_2CH_3$ | Br | 4-Cl |
| $CH_2CH_3$ | $CH_2CH_3$ | Br | 5-$CH_3$ |
| $CH_2CH_3$ | $CH_2CH_3$ | H | H |
| $CH_3$ | $CH(CH_3)_2$ | H | H |

TABLE IVd

| JOCH$_2$Q | | | |
|---|---|---|---|
| J is J-3 | | | |
| Q is Q-6 | | | |
| R$_9$ is in the 3 position | | | |
| $R_7$ | $R_8$ | $R_9$ | $R_{10}$ |
| $CH_3$ | $CH_3$ | Br | H |
| $CH_3$ | $CH_2CH_3$ | Br | H |
| $CH_3$ | $CH(CH_3)_2$ | Br | H |
| $CH_3$ | $CH(CH_3)_2$ | F | H |
| $CH_3$ | $CH(CH_3)_2$ | Cl | H |
| $CH_3$ | $CH(CH_3)_2$ | $CH_3$ | H |
| $CH_3$ | $CH(CH_3)_2$ | $CH_2CH_3$ | H |
| $CH_2CH_3$ | $CH_2CH_3$ | F | H |
| $CH_2CH_3$ | $CH_2CH_3$ | Cl | H |
| $CH_2CH_3$ | $CH_2CH_3$ | Br | H |
| $CH_2CH_3$ | $CH_2CH_3$ | $CH_3$ | H |
| $CH_2CH_3$ | $CH_2CH_3$ | $CH_2CH_3$ | H |
| $CH_2CH_3$ | $CH_2CH_3$ | Br | 5-F |
| $CH_2CH_3$ | $CH_2CH_3$ | Br | 5-Cl |
| $CH_2CH_3$ | $CH_2CH_3$ | Br | 5-$CH_3$ |
| $CH_3$ | $CH(CH_3)_2$ | H | H |
| $CH_2CH_3$ | $CH_2CH_3$ | H | H |

TABLE IVe

| JOCH$_2$Q | | | |
|---|---|---|---|
| J is J-3 | | | |
| Q is Q-7 | | | |
| $R_9$ is in the 4 position | | | |
| $R_7$ | $R_8$ | $R_9$ | $R_{10}$ |
| $CH_3$ | $CH_3$ | Br | H |
| $CH_3$ | $CH_2CH_3$ | Br | H |
| $CH_3$ | $CH(CH_3)_2$ | Br | H |
| $CH_3$ | $CH(CH_3)_2$ | F | H |
| $CH_3$ | $CH(CH_3)_2$ | Cl | H |
| $CH_3$ | $CH(CH_3)_2$ | $CH_3$ | H |
| $CH_3$ | $CH(CH_3)_2$ | $CH_2CH_3$ | H |
| $CH_2CH_3$ | $CH_2CH_3$ | F | H |
| $CH_2CH_3$ | $CH_2CH_3$ | Cl | H |
| $CH_2CH_3$ | $CH_2CH_3$ | Br | H |
| $CH_2CH_3$ | $CH_2CH_3$ | $CH_3$ | H |
| $CH_2CH_3$ | $CH_2CH_3$ | $CH_2CH_3$ | H |
| $CH_2CH_3$ | $CH_2CH_3$ | Br | 5-F |
| $CH_2CH_3$ | $CH_2CH_3$ | Br | 5-Cl |
| $CH_2CH_3$ | $CH_2CH_3$ | Br | 5-$CH_3$ |
| $CH_2CH_3$ | $CH_2CH_3$ | H | H |
| $CH_3$ | $CH(CH_3)_2$ | H | H |

TABLE IVf

| $JOCH_2Q$ | | |
|---|---|---|
| J is J-3 | | |
| Q is Q-8 | | |
| $R_9$ is in the 5 position | | |
| $R_7$ | $R_8$ | $R_9$ |
| $CH_3$ | $CH_3$ | Br |
| $CH_3$ | $CH_2CH_3$ | Br |
| $CH_3$ | $CH(CH_3)_2$ | Br |
| $CH_3$ | $CH(CH_3)_2$ | F |
| $CH_3$ | $CH(CH_3)_2$ | Cl |
| $CH_3$ | $CH(CH_3)_2$ | $CH_3$ |
| $CH_3$ | $CH(CH_3)_2$ | $CH_2CH_3$ |
| $CH_2CH_3$ | $CH_2CH_3$ | F |
| $CH_2CH_3$ | $CH_2CH_3$ | Cl |
| $CH_2CH_3$ | $CH_2CH_3$ | Br |
| $CH_2CH_3$ | $CH_2CH_3$ | $CH_3$ |
| $CH_2CH_3$ | $CH_2CH_3$ | $CH_2CH_3$ |
| $CH_2CH_3$ | $CH_2CH_3$ | Br |
| $CH_2CH_3$ | $CH_2CH_3$ | Br |
| $CH_2CH_3$ | $CH_2CH_3$ | Br |
| $CH_3$ | $CH(CH_3)_2$ | H |
| $CH_2CH_3$ | $CH_2CH_3$ | H |

TABLE IVg

| JOCH$_2$Q | | | |
|---|---|---|---|
| J is J-3 | | | |
| Q is Q-9 | | | |
| $R_9$ is in the 4 position | | | |
| $R_7$ | $R_8$ | $R_9$ | $R_{10}$ |
| $CH_3$ | $CH_3$ | Br | H |
| $CH_3$ | $CH_2CH_3$ | Br | H |
| $CH_3$ | $CH(CH_3)_2$ | Br | H |
| $CH_3$ | $CH(CH_3)_2$ | F | H |
| $CH_3$ | $CH(CH_3)_2$ | Cl | H |
| $CH_3$ | $CH(CH_3)_2$ | $CH_3$ | H |
| $CH_3$ | $CH(CH_3)_2$ | $CH_2CH_3$ | H |
| $CH_2CH_3$ | $CH_2CH_3$ | F | H |
| $CH_2CH_3$ | $CH_2CH_3$ | Cl | H |
| $CH_2CH_3$ | $CH_2CH_3$ | Br | H |
| $CH_2CH_3$ | $CH_2CH_3$ | $CH_3$ | H |
| $CH_2CH_3$ | $CH_2CH_3$ | $CH_2CH_3$ | H |
| $CH_2CH_3$ | $CH_2CH_3$ | Br | 5-F |
| $CH_2CH_3$ | $CH_2CH_3$ | Br | 5-Cl |
| $CH_2CH_3$ | $CH_2CH_3$ | Br | 5-$CH_3$ |
| $CH_2CH_3$ | $CH_2CH_3$ | H | H |
| $CH_3$ | $CH(CH_3)_2$ | H | H |

TABLE IVh

| JOCH$_2$Q | | | |
|---|---|---|---|
| J is J-3 | | | |
| Q is Q-10 | | | |
| $R_9$ is in the 5 position | | | |
| $R_7$ | $R_8$ | $R_9$ | $R_{10}$ |
| $CH_3$ | $CH_3$ | Br | H |
| $CH_3$ | $CH_2CH_3$ | Br | H |
| $CH_3$ | $CH(CH_3)_2$ | Br | H |
| $CH_3$ | $CH(CH_3)_2$ | F | H |
| $CH_3$ | $CH(CH_3)_2$ | Cl | H |
| $CH_3$ | $CH(CH_3)_2$ | $CH_3$ | H |
| $CH_3$ | $CH(CH_3)_2$ | $CH_2CH_3$ | H |
| $CH_2CH_3$ | $CH_2CH_3$ | F | H |
| $CH_2CH_3$ | $CH_2CH_3$ | Cl | H |
| $CH_2CH_3$ | $CH_2CH_3$ | Br | H |
| $CH_2CH_3$ | $CH_2CH_3$ | $CH_3$ | H |
| $CH_2CH_3$ | $CH_2CH_3$ | $CH_2CH_3$ | H |
| $CH_2CH_3$ | $CH_2CH_3$ | Br | 2-F |
| $CH_2CH_3$ | $CH_2CH_3$ | Br | 2-Cl |
| $CH_2CH_3$ | $CH_2CH_3$ | Br | 2-$CH_3$ |
| $CH_2CH_3$ | $CH_2CH_3$ | H | H |
| $CH_3$ | $CH(CH_3)_2$ | H | H |

TABLE IVi

| $JOCH_2Q$ | | | |
|---|---|---|---|
| J is J-3 | | | |
| Q is Q-11 | | | |
| $R_9$ is in the 4 position | | | |
| $R_7$ | $R_8$ | $R_9$ | $R_{10}$ |
| $CH_3$ | $CH_3$ | Br | H |
| $CH_3$ | $CH_2CH_3$ | Br | H |
| $CH_3$ | $CH(CH_3)_2$ | Br | H |
| $CH_3$ | $CH(CH_3)_2$ | F | H |
| $CH_3$ | $CH(CH_3)_2$ | Cl | H |
| $CH_3$ | $CH(CH_3)_2$ | $CH_3$ | H |
| $CH_3$ | $CH(CH_3)_2$ | $CH_2CH_3$ | H |
| $CH_2CH_3$ | $CH_2CH_3$ | F | H |
| $CH_2CH_3$ | $CH_2CH_3$ | Cl | H |
| $CH_2CH_3$ | $CH_2CH_3$ | Br | H |
| $CH_2CH_3$ | $CH_2CH_3$ | $CH_3$ | H |
| $CH_2CH_3$ | $CH_2CH_3$ | $CH_2CH_3$ | H |
| $CH_2CH_3$ | $CH_2CH_3$ | Br | 2-F |
| $CH_2CH_3$ | $CH_2CH_3$ | Br | 2-Cl |
| $CH_2CH_3$ | $CH_2CH_3$ | Br | 2-$CH_3$ |
| $CH_2CH_3$ | $CH_2CH_3$ | H | H |
| $CH_3$ | $CH(CH_3)_2$ | H | H |

TABLE IVj

| $JOCH_2Q$ | | | |
|---|---|---|---|
| J is J-3 | | | |
| Q is Q-12 | | | |
| $R_9$ is in the 5 position | | | |
| $R_7$ | $R_8$ | $R_9$ | $R_{10}$ |
| $CH_3$ | $CH_3$ | Br | H |
| $CH_3$ | $CH_2CH_3$ | Br | H |
| $CH_3$ | $CH(CH_3)_2$ | Br | H |
| $CH_3$ | $CH(CH_3)_2$ | F | H |
| $CH_3$ | $CH(CH_3)_2$ | Cl | H |
| $CH_3$ | $CH(CH_3)_2$ | $CH_3$ | H |
| $CH_3$ | $CH(CH_3)_2$ | $CH_2CH_3$ | H |
| $CH_2CH_3$ | $CH_2CH_3$ | F | H |
| $CH_2CH_3$ | $CH_2CH_3$ | Cl | H |
| $CH_2CH_3$ | $CH_2CH_3$ | Br | H |
| $CH_2CH_3$ | $CH_2CH_3$ | $CH_3$ | H |
| $CH_2CH_3$ | $CH_2CH_3$ | $CH_2CH_3$ | H |
| $CH_2CH_3$ | $CH_2CH_3$ | H | H |
| $CH_3$ | $CH(CH_3)_2$ | H | H |

TABLE IVk

| JOCH2Q | | | |
|---|---|---|---|
| J is J-3 | | | |
| Q is Q-13 | | | |
| $R_9$ is in the 4 position | | | |
| $R_7$ | $R_8$ | $R_9$ | $R_{10}$ |
| $CH_3$ | $CH_3$ | Br | H |
| $CH_3$ | $CH_2CH_3$ | Br | H |
| $CH_3$ | $CH(CH_3)_2$ | Br | H |
| $CH_3$ | $CH(CH_3)_2$ | F | H |
| $CH_3$ | $CH(CH_3)_2$ | Cl | H |
| $CH_3$ | $CH(CH_3)_2$ | $CH_3$ | H |
| $CH_3$ | $CH(CH_3)_2$ | $CH_2CH_3$ | H |
| $CH_2CH_3$ | $CH_2CH_3$ | F | H |
| $CH_2CH_3$ | $CH_2CH_3$ | Cl | H |
| $CH_2CH_3$ | $CH_2CH_3$ | Br | H |
| $CH_2CH_3$ | $CH_2CH_3$ | $CH_3$ | H |
| $CH_2CH_3$ | $CH_2CH_3$ | $CH_2CH_3$ | H |
| $CH_2CH_3$ | $CH_2CH_3$ | Br | 2-F |
| $CH_2CH_3$ | $CH_2CH_3$ | Br | 2-Cl |
| $CH_2CH_3$ | $CH_2CH_3$ | Br | 2-$CH_3$ |
| $CH_2CH_3$ | $CH_2CH_3$ | H | H |
| $CH_3$ | $CH(CH_3)_2$ | H | H |

TABLE IVI

| JOCH$_2$Q | | | |
|---|---|---|---|
| J is J-3 | | | |
| Q is Q-14 | | | |
| $R_9$ is in the 5 position | | | |
| $R_7$ | $R_8$ | $R_9$ | $R_{10}$ |
| $CH_3$ | $CH_3$ | Br | H |
| $CH_3$ | $CH_2CH_3$ | Br | H |
| $CH_3$ | $CH(CH_3)_2$ | Br | H |
| $CH_3$ | $CH(CH_3)_2$ | F | H |
| $CH_3$ | $CH(CH_3)_2$ | Cl | H |
| $CH_3$ | $CH(CH_3)_2$ | $CH_3$ | H |
| $CH_3$ | $CH(CH_3)_2$ | $CH_2CH_3$ | H |
| $CH_2CH_3$ | $CH_2CH_3$ | F | H |
| $CH_2CH_3$ | $CH_2CH_3$ | Cl | H |
| $CH_2CH_3$ | $CH_2CH_3$ | Br | H |
| $CH_2CH_3$ | $CH_2CH_3$ | $CH_3$ | H |
| $CH_2CH_3$ | $CH_2CH_3$ | $CH_2CH_3$ | H |
| $CH_2CH_3$ | $CH_2CH_3$ | Br | 2-F |
| $CH_2CH_3$ | $CH_2CH_3$ | Br | 2-Cl |
| $CH_2CH_3$ | $CH_2CH_3$ | Br | 2-$CH_3$ |
| $CH_2CH_3$ | $CH_2CH_3$ | H | H |
| $CH_3$ | $CH(CH_3)_2$ | H | H |

## Formulations

The method of this invention can be conveniently carried out by formulating a compound of Formula I in the conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The herbicidal formulations of the invention comprise 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

| | Weight Percent* | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0 °C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science", John Wiley and Sons, Inc., New York, 1961, pp. 81-96; and

J. D. Fryer and S. A. Evans, "Weed Control Handbook", 5th Ed., Blackwell Scientific Publications, Oxford, 1968, pp. 101-103.

In the following Examples, all parts are by weight unless otherwise indicated.

| Example A | |
|---|---|
| Wettable Powder | |
| 6-endo-[(2,6-difluorophenyl)methoxy]-3,3-diethyl-1-methyl-2-oxabicyclo[2.2.2]octane | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

| Example B | |
|---|---|
| Granule | |
| Wettable Powder of Example A | 5% |
| attapulgite granules (U.S.S. 20 to 40 mesh; 0.84-0.42 mm) | 95% |

A slurry of wettable powder containing 25% solids is sprayed on the surface of attapulgite granules in a double-cone blender. The granules are dried and packaged.

| Example C | |
|---|---|
| Extruded Pellet | |
| 6-*endo*-[(2,6-difluorophenyl)methoxy]-3,3-diethyl-1-methyl-2-oxabicyclo[2.2.2]octane | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

| Example D | |
|---|---|
| Low Strength Granule | |
| 6-*endo*-[(2,6-difluorophenyl)methoxy]-3,3-diethyl-1-methyl-2-oxabicyclo[2.2.2]octane | 0.1% |
| attapulgite granules (U.S.S. 20 to 40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

| Example E | |
|---|---|
| Low Strength Granule | |
| 6-*endo*-[(2,6-difluorophenyl)methoxy]-3,3-diethyl-1-methyl-2-oxabicyclo[2.2.2]octane | 1% |
| N,*N*-dimethylformamide | 9% |
| attapulgite granules (U.S.S. 20 to 40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

| Example F | |
|---|---|
| Wettable Powder | |
| 6-*endo*-[(2,6-difluorophenyl)methoxy]-3,3-diethyl-1-methyl-2-oxabicyclo[2.2.2]octane | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

| Example G | |
|---|---|
| Emulsifiable Concentrate | |
| 6-endo-[(2,6-difluorophenyl)methoxy]-3,3-diethyl-1-methyl-2-oxabicyclo[2.2.2]octane<br>blend of polyalcohol carboxylic esters and oil soluble petroleum sulfonates<br>xylene | 35%<br>6%<br>59% |

The ingredients are combined and filtered to remove undissolved solids. The product can be used directly, extended with oils, or emulsified in water.

| Example H | |
|---|---|
| Dust | |
| 6-endo-[(2,6-difluorophenyl)methoxy]-3,3-diethyl-1-methyl-2-oxabicyclo[2.2.2]octane<br>attapulgite<br>Pyrophyllite | 10%<br>10%<br>80% |

The active ingredient is blended with attapulgite and then passed through a hammer-mill to produce particles substantially all below 200 microns. The ground concentrate is then blended with powdered pyrophyllite until homogeneous.

| Example I | |
|---|---|
| Wettable Powder | |
| 6-endo-[(2,6-difluorophenyl)methoxy]-3,3-diethyl-1-methyl-2-oxabicyclo[2.2.2]octane<br>sodium alkylnaphthalenesulfonate<br>sodium ligninsulfonate<br>low viscosity methyl cellulose<br>attapulgite | 20%<br>4%<br>4%<br>3%<br>69% |

The ingredients are thoroughly blended. After grinding in a hammer-mill to produce particles essentially all below 100 microns, the material is reblended and sifted through a U.S.S. No. 50 sieve (0.3 mm opening) and packaged.

## Utility

Test results indicate that compounds of this invention are active postemergence and, in particular, preemergence herbicides. Many compounds of this invention are useful for the control of selected grass and broadleaf weeds with tolerance to important agronomic crops such as barley (Hordeum vulgare), corn (Zea mays), cotton (Gossypium hirsutum), rape (Brassica napus), rice (Oryza sativa), sorghum (Sorghum bicolor), soybean (Glycine max), sugar beet (Beta vulgaris), and wheat (Triticum aestivum). Grass weeds controlled include, but are not limited to, barnyardgrass (Echinochloa crus-galli), blackgrass (Alopecurus myosuroides), crabgrass (Digitaria spp.), foxtail (Setaria spp.), and johnsongrass (Sorghum halepense). Several compounds in this invention are particularly useful for the control of barnyardgrass in upland and paddy rice. Utility in paddy rice includes both direct-seeded and transplanted paddy rice.

Several compounds in this invention have utility in non-crop areas where selected weed control is desired, such as around storage tanks, parking lots, drive-in theaters, billboards, highways, and railroad structures. These compounds are also useful in fallow areas of crop production such as in wheat and barley and in plantation crops such as palm, banana, citrus, rubber, etc. Alternatively, these compounds may be useful to modify plant growth.

The effective amount for compounds of this invention is determined by a number of factors. These

factors include: formulation selected, method of application, amount of vegetation present, growing conditions, etc. In general terms, the effective amount of the subject compounds is applied at rates from 0.01 to 20 kg/ha with a preferred rate range of from 0.02 to 1 kg/ha. One skilled in the art can easily determine rates needed for the desired level of weed control.

Compounds of this invention may be used alone or in combination with other commercial herbicides, insecticides, or fungicides. The following list exemplifies some of the herbicides suitable for use in mixtures. A combination of compounds from this invention with one or more of the following herbicides may be particularly useful for weed control.

| Common Name | Chemical Name |
|---|---|
| acetochlor | 2-chloro-N-(ethoxymethyl)-N-(2-ethyl-6-methylphenyl)acetamide |
| acifluorfen | 5-[2-chloro-4-(trifluoromethyl)-phenoxy]-2-nitrobenzoic acid |
| acrolein | 2-propenal |
| alachlor | 2-chloro-N-(2,6-diethylphenyl)-N-(methoxymethyl)acetamide |
| anilofos | S-4-chloro-N-isopropylcarbaniloyl-methyl-O,O-dimethyl phosphorodithioate |

| Common Name | Chemical Name |
| --- | --- |
| ametryn | N-ethyl-N'-(1-methylethyl)-6-(methylthio)-1,3,5-triazine-2,4-diamine |
| amitrole | 1H-1,2,4-triazol-3-amine |
| AMS | ammonium sulfamate |
| asulam | methyl [(4-aminophenyl)sulfonyl]-carbamate |
| atrazine | 6-chloro-N-ethyl-N'-(1-methylethyl)-1,3,5-triazine-2,4-diamine |
| barban | 4-chloro-2-butynyl 3-chlorocarbamate |
| benefin | N-butyl-N-ethyl-2,6-dinitro-4-(trifluoromethyl)benzenamine |
| bensulfuron methyl | 2-[[[[[(4,6-dimethoxy-2-pyrimidinyl)amino]methylcarbonyl]-amino]sulfonyl]methyl]benzoic acid, methyl ester |
| bensulide | O,O-bis(1-methylethyl) S-[2-[(phenylsulfonyl)amino]-ethyl]phosphorodithioate |
| bentazon | 3-(1-methylethyl)-(1H)-2,1,3-benzothiadiazin-4(3H)-one, 2,2-dioxide |
| benzofluor | N-[4-(ethylthio)-2-(trifluoromethyl)phenyl]methanesulfonamide |
| benzoylprop | N-benzoyl-N-(3,4-dichlorophenyl)-DL-alanine |
| bifenox | methyl 5-(2,4-dichlorophenoxy)-2-nitrobenzoate |
| bromacil | 5-bromo-6-methyl-3-(1-methylpropyl)-2,4(1H,3H)pyrimidinedione |
| bromoxynil | 3,5-dibromo-4-hydroxybenzonitrile |

| Common Name | Chemical Name |
|---|---|
| butachlor | N-(butoxymethyl)-2-chloro-N-(2,6-diethylphenyl)acetamide |
| buthidazole | 3-[5-(1,1-dimethylethyl)-1,3,4-thiadiazol-2-yl]-4-hydroxy-1-methyl-2-imidazolidinone |
| butralin | 4-(1,1-dimethylethyl)-N-(1-methylpropyl)-2,6-dinitrobenzenamine |
| butylate | S-ethyl bis(2-methylpropyl)-carbamothioate |
| cacodylic acid | dimethyl arsinic oxide |
| CDAA | 2-chloro-N,N-di-2-propenylacetamide |
| CDEC | 2-chloroallyl diethyldithiocarbamate |
| CGA 142,464 | 3-(4,6-dimethoxy-1,3,5-triazin-2-yl)-1-[2-(2-methoxyethoxy)-phenylsulfonyl]-urea |
| chloramben | 3-amino-2,5-dichlorobenzoic acid |
| chlorbromuron | 3-(4-bromo-3-chlorophenyl)-1-methoxy-1-methylurea |
| chlorimuron ethyl | 2-[[[[(4-chloro-6-methoxy-2-pyrimidinyl)ethylamino]carbonyl]-amino]sulfonyl]benzoic acid, ethyl ester |
| chlormethoxynil | 2,4-dichlorophenyl 4-nitro-3-methoxyphenyl ether |
| chlornitrofen | 2,4,6-trichlorophenyl-4-nitrophenyl ether |
| chloroxuron | N'-[4-(4-chlorophenoxy)phenyl]-N,N-dimethylurea |
| chlorpropham | 1-methylethyl 3-chlorophenylcarbamate |
| chlorsulfuron | 2-chloro-N-[[(4-methoxy-6-methyl-1,3,5-triazin-2-yl)amino]carbonyl]benzenesulfonamide |

| Common Name | Chemical Name |
|---|---|
| chlortoluron | N'-(3-chloro-4-methylphenyl)-N,N-dimethylurea |
| cinmethylin | exo-1-methyl-4-(1-methylethyl)-2-[(2-methylphenyl)methoxy]-7-oxabicyclo-[2.2.1]heptane |
| clethodim | (E,E)-(±)-2-[1-[[(3-chloro-2-propenyl)-oxy]imino]propyl]-5-[2-(ethylthio)-propyl]-3-hydroxy-2-cyclohexen-1-one |
| clomazone | 2-[(2-chlorophenyl)methyl]-4,4-dimethyl-3-isoxazolidinone |
| cloproxydim | (E,E)-2-[1-[[(3-chloro-2-propenyl)oxy)-imino]butyl]-5-[2-(ethylthio)propyl]-3-hydroxy-2-cyclohexen-1-one |
| clopyralid | 3,6-dichloro-2-pyridinecarboxylic acid |
| CMA | calcium salt of MAA |
| cyanazine | 2-[[4-chloro-6-(ethylamino)-1,3,5-tri-azin-2-yl]amino]-2-methylpropanenitrile |
| cycloate | S-ethyl cyclohexylethylcarbamothioate |
| cycluron | 3-cyclooctyl-1,1-dimethylurea |
| cyperquat | 1-methyl-4-phenylpyridinium |
| cyprazine | 2-chloro-4-(cyclopropylamino)-6-(iso-propylamino)-s-triazine |
| cyprazole | N-[5-(2-chloro-1,1-dimethylethyl)-1,3,4-thiadiazol-2-yl]cyclopropanecarbox-amide |
| cypromid | 3',4'-dichlorocyclopropanecarboxanilide |
| dalapon | 2,2-dichloropropanoic acid |
| dazomet | tetrahydro-3,5-dimethyl-2H-1,3,5-thia-diazine-2-thione |
| DCPA | dimethyl 2,3,5,6-tetrachloro-1,4-benzene-dicarboxylate |

| Common Name | Chemical Name |
|---|---|
| desmediphan | ethyl [3-[[(phenylamino)carbonyl]oxy]-phenyl]carbamate |
| desmetryn | 2-(isopropylamino)-4-(methylamino)-6-(methylthio)-s-triazine |
| diallate | S-(2,3-dichloro-2-propenyl)bis(1-methylethyl)carbamothioate |
| dicamba | 3,6-dichloro-2-methoxybenzoic acid |
| dichlobenil | 2,6-dichlorobenzonitrile |
| dichlorprop | (±)-2-(2,4-dichlorophenoxy)propanoic acid |
| dichlofop | (±)-2-[4-(2,4-dichlorophenoxy)phenoxy]-propanoic acid, methyl ester |
| diethatyl | N-(chloroacetyl)-N-(2,6-diethylphenyl)-glycine |
| difenzoquat | 1,2-dimethyl-3,5-diphenyl-1H-pyrazolium |
| dimepiperate | S-1-methyl-1-phenylethylpiperidine-1-carbothioate |
| dinitramine | $N^3$,$N^3$-diethyl-2,4-dinitro-6-(trifluoro-methyl)-1,3-benzenediamine |
| dinoseb | 2-(1-methylpropyl)-4,6-dinitrophenol |
| diphenamid | N,N-dimethyl-α-phenylbenzeneacetamide |
| dipropetryn | 6-(ethylthio)-N,N'-bis(1-methylethyl)-1,3,5-triazine-2,4-diamine |
| diquat | 6,7-dihydrodipyrido[1,2-a:2',1'-c]-pyrazinedium ion |
| diuron | N'-(3,4-dichlorophenyl)-N,N-dimethylurea |
| DNOC | 2-methyl-4,6-dinitrophenol |
| DPX-M6316 | 3-[[[[(4-methoxy-6-methyl-1,3,5-triazin-2-yl)amino]carbonyl]amino]sulfonyl]-2-thiophenecarboxylic acid, methyl ester |

| Common Name | Chemical Name |
|---|---|
| DSMA | disodium salt of MAA |
| dymron | N-(4-methylphenyl)-N'-(1-methyl-1-phenylethyl)urea |
| endothall | 7-oxabicyclo[2.2.1]heptane-2,3-dicarboxylic acid |
| EPTC | S-ethyl dipropylcarbamothioate |
| esprocarb (SC2957) | S-benzyl-N-ethyl-N-(1,2-dimethyl)-propyl)thiolcarbamate |
| ethalfluralin | N-ethyl-N-(2-methyl-2-propenyl)-2,6-dinitro-4-(trifluoromethyl)-benzenamine |
| ethofumesate | (±)-2-ethoxy-2,3-dihydro-3,3-dimethyl-5-benzofuranyl methanesulfonate |
| Express® | 2-[[[[N-(4-methoxy-6-methyl-1,3,5-triazine-2-yl)-N-methylamino]-carbonyl]amino]sulfonyl]benzoic acid, methyl ester |
| fenac | 2,3,6-trichlorobenzeneacetic acid |
| fenoxaprop | (±)-2-[4-[(6-chloro-2-benzoxazolyl)oxy]-phenoxy]propanoic acid |
| fenuron | N,N-dimethyl-N'-phenylurea |
| fenuron TCA | Salt of fenuron and TCA |
| flamprop | N-benzoyl-N-(3-chloro-4-fluorophenyl)-DL-alanine |
| fluazifop | (±)-2-[4-[[5-(trifluoromethyl)-2-pyridinyl]oxy]phenoxy]propanoic acid |
| fluazifop-P | (R)-2-[4-[[5-(trifluoromethyl)-2-pyridinyl]oxy]phenoxy]propanoic acid |
| fluchloralin | N-(2-chloroethyl)-2,6-dinitro-N-propyl-4-(trifluoromethyl)benzenamine |
| fluometuron | N,N-dimethyl-N'-[3-(trifluoromethyl)-phenyl]urea |

| Common Name | Chemical Name |
|---|---|
| fluorochloridone | 3-chloro-4-(chloromethyl)-1-[3-(trifluoromethyl)phenyl]-2-pyrrolidinone |
| fluorodifen | p-nitrophenyl α,α,α-trifluoro-2-nitro-p-tolyl ether |
| fluoroglycofen | carboxymethyl 5-[2-chloro-4-(trifluoromethyl)phenoxy]-2-nitrobenzoate |
| fluridone | 1-methyl-3-phenyl-5-[3-(trifluoromethyl)phenyl]-4(1H)-pyridinone |
| fomesafen | 5-[2-chloro-4-(trifluoromethyl)phenoxy]-N-(methylsulfonyl)-2-nitrobenzamide |
| fosamine | ethyl hydrogen (aminocarbonyl)-phosphate |
| glyphosate | N-(phosphonomethyl)glycine |
| haloxyfop | 2-[4-[[3-chloro-5-(trifluoromethyl)-2-pyridinyl]oxy]phenoxy]propanoic acid |
| hexaflurate | potassium hexafluoroarsenate |
| hexazinone | 3-cyclohexyl-6-(dimethylamino)-1-methyl-1,3,5-triazine-2,4(1H,3H)-dione |
| imazamethabenz | 6-(4-isopropyl-4-methyl-5-oxo-2-imidazolin-2-yl)-m-toluic acid, methyl ester and 6-(4-isopropyl-4-methyl-5-oxo-2-imidazolin-2-yl)-p-toluic acid, methyl ester |
| imazapyr | (±)-2-[4,5-dihydro-4-methyl-4-(1-methylethyl)-5-oxo-1H-imidazol-2-yl]-3-pyridinecarboxylic acid |
| imazaquin | 2-[4,5-dihydro-4-methyl-4-(1-methylethyl)-5-oxo-1H-imidazol-2-yl]-3-quinolinecarboxylic acid |
| imazethapyr | (±)-2-[4,5-dihydro-4-methyl-4-(1-methylethyl)-5-oxo-1H-imidazol-2-yl]-5-ethyl-3-pyridinecarboxylic acid |
| ioxynil | 4-hydroxy-3,5-diiodobenzonitrile |

| Common Name | Chemical Name |
|---|---|
| isopropalin | 4-(1-methylethyl)-2,6-dinitro-N,N-dipropylbenzenamine |
| isoproturon | N-(4-isopropylphenyl)-N',N'-dimethylurea |
| isouron | N'-[5-(1,1-dimethylethyl)-3-isoxazolyl]-N,N-dimethylurea |
| isoxaben | N-[3-(1-ethyl-1-methylpropyl)-5-isoxazolyl]-2,6-dimethoxybenzamide |
| karbutilate | 3-[[(dimethylamino)carbonyl]amino]-phenyl-(1,1-dimethylethyl)carbamate |
| lactofen | (±)-2-ethoxy-1-methyl-2-oxoethyl 5-[2-chloro-4-(trifluoromethyl)phenoxy]-2-nitrobenzoate |
| lenacil | 3-cyclohexyl-6,7-dihydro-1H-cyclopenta-pyrimidine-2,4(3H,5H)-dione |
| linuron | N'-(3,4-dichlorophenyl)-N-methoxy-N-methylurea |
| MAA | methylarsonic acid |
| MAMA | monoammonium salt of MAA |
| MCPA | (4-chloro-2-methylphenoxy)acetic acid |
| MCPB | 4-(4-chloro-2-methylphenoxy)butanoic acid |
| MON 7200 | S,S-dimethyl-2-(difluoromethyl)-4-(2-methylpropyl)-6-(trifluoromethyl)-3,5-pyridinedicarbothionate |
| mecoprop | (±)-2-(4-chloro-2-methylphenoxy)-propanoic acid |
| mefenacet | 2-(2-benzothiazolyloxy-N-methyl-N-phenylacetamide |
| mefluidide | N-[2,4-dimethyl-5-[[(trifluoromethyl)-sulfonyl]amino]phenyl]acetamide |
| methal-propalin | N-(2-methyl-2-propenyl)-2,6-dinitro-N-propyl-4-(trifluoromethyl)benzenamide |

| Common Name | Chemical Name |
|---|---|
| methabenz-thiazuron | 1,3-dimethyl-3-(2-benzothiazolyl)urea |
| metham | methylcarbamodithioic acid |
| methazole | 2-(3,4-dichlorophenyl)-4-methyl-1,2,4-oxadiazolidine-3,5-dione |
| methoxuron | N'-(3-chloro-4-methoxyphenyl)-N,N-dimethylurea |
| metolachlor | 2-chloro-N-(2-ethyl-6-methylphenyl)-N-(2-methoxy-1-methylethyl)acetamide |
| metribuzin | 4-amino-6-(1,1-dimethylethyl)-3-(methylthio)-1,2,4-triazin-5(4H)-one |
| metsulfuron methyl | 2-[[[[(4-methoxy-6-methyl-1,3,5-triazin-2-yl)amino]carbonyl]-amino]sulfonyl]benzoic acid, methyl ester |
| MH | 1,2-dihydro-3,6-pyridazinedione |
| molinate | S-ethyl hexahydro-1H-azepine-1-carbothioate |
| monolinuron | 3-(p-chlorophenyl)-1-methoxy-1-methylurea |
| monuron | N'-(4-chlorophenyl)-N,N-dimethylurea |
| monuron TCA | Salt of monuron and TCA |
| MSMA | monosodium salt of MAA |
| napropamide | N,N-diethyl-2-(1-naphthalenyloxy)-propanamide |
| naptalam | 2-[(1-naphthalenylamino)carbonyl]-benzoic acid |
| neburon | 1-butyl-3-(3,4-dichlorophenyl)-1-methylurea |
| nitralin | 4-(methylsulfonyl)-2,6-dinitro-N,N-dipropylaniline |

| Common Name | Chemical Name |
|---|---|
| nitrofen | 2,4-dichloro-1-(4-nitrophenoxy)benzene |
| nitrofluorfen | 2-chloro-1-(4-nitrophenoxy)-4-(trifluoromethyl)benzene |
| norea | N,N-dimethyl-N'-(octahydro-4,7-methano-1H-inden-5-yl)urea 3aα,-4α,5α,7α,7aα-isomer |
| norflurazon | 4-chloro-5-(methylamino)-2-[3-(trifluoromethyl)phenyl]-3(2H)-pyridazinone |
| oryzalin | 4-(dipropylamino)-3,5-dinitrobenzenesulfonamide |
| oxadiazon | 3-[2,4-dichloro-5-(1-methylethoxy)-phenyl]-5-(1,1-dimethylethyl)-1,3,4-oxadiazol-2(3H)-one |
| oxyfluorfen | 2-chloro-1-(3-ethoxy-4-nitrophenoxy)-4-(trifluoromethyl)benzene |
| paraquat | 1,1'-dimethyl-4,4'-dipyridinium ion |
| pebulate | S-propyl butylethylcarbamothioate |
| pendimethalin | N-(1-ethylpropyl)-3,4-dimethyl-2,6-dinitrobenzenamine |
| perfluidone | 1,1,1-trifluoro-N-[2-methyl-4-(phenylsulfonyl)phenyl]methanesulfonamide |
| phenmedipham | 3-[(methoxycarbonyl)amino]phenyl (3-methylphenyl)carbamate |
| picloram | 4-amino-3,5,6-trichloro-2-pyridinecarboxylic acid |
| PPG-1013 | 5-[2-chloro-4-(trifluoromethyl)-phenoxy]-2-nitroacetophenone oxime-O-acetic acid, methyl ester |
| pretilachlor | α-chloro-2,6-diethyl-N-(2-propoxyethyl)acetanilide |

| Common Name | Chemical Name |
|---|---|
| procyazine | 2-[[4-chloro-6-(cyclopropylamino)-1,3,5-triazine-2-yl]amino]-2-methylpropanenitrile |
| profluralin | N-(cyclopropylmethyl)-2,6-dinitro-N-propyl-4-(trifluoromethyl)benzenamine |
| prometon | 6-methoxy-N,N'-bis(1-methylethyl)-1,3,5-triazine-2,4-diamine |
| prometryn | N,N'-bis(1-methylethyl)-6-(methylthio)-1,3,5-triazine-2,4-diamine |
| pronamide | 3,5-dichloro-N-(1,1-dimethyl-2-propynyl)benzamide |
| propachlor | 2-chloro-N-(1-methylethyl)-N-phenylacetamide |
| propanil | N-(3,4-dichlorophenyl)propanamide |
| propazine | 6-chloro-N,N'-bis(1-methylethyl)-1,3,5-triazine-2,4-diamine |
| propham | 1-methylethyl phenylcarbamate |
| prosulfalin | N-[[4-(dipropylamino)-3,5-dinitrophenyl]sulfonyl]-S,S-dimethylsulfilimine |
| prynachlor | 2-chloro-N-(1-methyl-2-propynyl)acetanilide |
| pyrazolate | 4-(2,4-dichlorobenzoyl)-1,3-dimethylpyrazol-5-yl-*p*-toluenesulphonate |
| pyrazon | 5-amino-4-chloro-2-phenyl-3(2H)-pyridazinone |
| pyrazosulfuron ethyl | ethyl S-[3-(4,6-dimethoxypyrimidin-2-yl)ureadosulfonyl]-1-methylpyrazole-4-carboxylate |
| quinclorac | 3,7-dichloro-8-quinoline carboxylic acid |
| quizalofop ethyl | (±)-2-[4-[(6-chloro-2-quinoxalinyl)-oxy]phenoxy]propanoic acid, ethyl ester |

| Common Name | Chemical Name |
|---|---|
| secbumeton | N-ethyl-6-methoxy-N'-(1-methylpropyl)-1,3,5-triazine-2,4-diamine |
| sethoxydim | 2-[1-(ethoxyimino)butyl]-5-[2-(ethylthio)propyl]-3-hydroxy-2-cyclohexen-1-one |
| siduron | N-(2-methylcyclohexyl)-N'-phenylurea |
| simazine | 6-chloro-N,N'-diethyl-1,3,5-triazine-2,4-diamine |
| SK-233 | 1-(α,α-dimethylbenzyl)-3-(4-methylphenyl)urea |
| sulfometuron methyl | 2-[[[[(4,6-dimethyl-2-pyrimidinyl)-amino]carbonyl]amino]sulfonyl]-benzoic acid, methyl ester |
| TCA | trichloroacetic acid |
| tebuthiuron | N-[5-(1,1-dimethylethyl)-1,3,4-thiadiazol-2-yl]-N,N'-dimethylurea |
| terbacil | 5-chloro-3-(1,1-dimethylethyl)-6-methyl-2,4(1H,3H)-pyrimidinedione |
| terbuchlor | N-(butoxymethyl)-2-chloro-N-[2-(1,1-dimethylethyl)-6-methylphenyl]-acetamide |
| terbuthylazine | 2-(tert-butylamino)-4-chloro-6-(ethylamino)-s-triazine |
| terbutol | 2,6-di-tert-butyl-p-tolyl methylcarbamate |
| terbutryn | N-(1,1-dimethylethyl)-N'-ethyl-6-(methylthio)-1,3,5-triazine-2,4-diamine |
| thiobencarb | S-[(4-chlorophenyl)methyl] diethylcarbamothioate |
| triallate | S-(2,3,3-trichloro-2-propenyl) bis(1-methylethyl)carbamothioate |

| Common Name | Chemical Name |
|---|---|
| triclopyr | [(3,5,6-trichloro-2-pyridinyl)-oxy]acetic acid |
| tridiphane | 2-(3,5-dichlorophenyl)-2-(2,2,2-trichloroethyl)oxirane |
| trifluralin | 2,6-dinitro-N,N-dipropyl-4-(tri-fluoromethyl)benzenamine |
| trimeturon | 1-(p-chlorophenyl)-2,3,3-trimethylpseu-dourea |
| 2,4-D | (2,4-dichlorophenoxy)acetic acid |
| 2,4-DB | 4-(2,4-dichlorophenoxy)butanoic acid |
| vernolate | S-propyl dipropylcarbamothioate |
| xylachlor | 2-chloro-N-(2,3-dimethylphenyl)-N-(1-methylethyl)acetamide |

Herbicidal properties of the subject compounds were discovered in a series of greenhouse tests. Test procedures and results follow.

## BIOLOGICAL TABLE 1

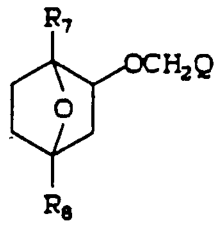

| CMPD | $R_7$ | $R_8$ | Q |
|---|---|---|---|
| 1 | $CH_3$ | $CH(CH_3)_2$ | Q-3 ($R_9$ = 2-Br, $R_{10}$ = H) |
| 2 | $CH_3$ | $CH(CH_3)_2$ | Q-3 ($R_9$ = 2-$CH_3$, $R_{10}$ = H) |
| 3 | $CH_3$ | $CH(CH_3)_2$ | Q-3 ($R_9$ = 2-$CH_2CH_3$, $R_{10}$ = H) |
| 4 | $CH_3$ | $CH(CH_3)_2$ | Q-3 ($R_9$ = H, $R_{10}$ = H) |
| 5 | $CH_3$ | $CH(CH_3)_2$ | Q-14 ($R_9$ = H, $R_{10}$ = H) |
| 6 | $CH_3$ | $CH(CH_3)_2$ | Q-2 ($R_9$ = H, $R_{10}$ = 6-F) |
| 7 | $CH_3$ | $CH(CH_3)_2$ | Q-14 ($R_9$ = 2-$CH_3$, $R_{10}$ = H) |
| 8 | $CH_3$ | $CH(CH_3)_2$ | Q-2 ($R_9$ = 6-$CH_3$, $R_{10}$ = H) |
| 9 | $CH_3$ | $CH(CH_3)_2$ | Q-5 ($R_9$ = 4-$CH_3$, $R_{10}$ = H) |
| 10 | $CH_3$ | $CH(CH_3)_2$ | Q-13 ($R_9$ = H, $R_{10}$ = 4-$CH_3$) |
| 11 | $CH_3$ | $CH(CH_3)_2$ | Q-2 ($R_9$ = H, $R_{10}$ = 6-Br) |
| 12 | $CH_3$ | $CH(CH_3)_2$ | Q-5 ($R_9$ = H, $R_{10}$ = H) |
| 13 | $CH_2CH_3$ | $CH_2CH_3$ | Q-2 ($R_9$ = H, $R_{10}$ = 6-F) |
| 14 | $CH_2CH_3$ | $CH_2CH_3$ | Q-2 ($R_9$ = H, $R_{10}$ = 6-$CH_3$) |
| 15 | $CH_2CH_3$ | $CH_2CH_3$ | Q-14 ($R_9$ = H, $R_{10}$ = H) |
| 17 | $CH_3$ | $CH(CH_3)_2$ | Q-3 ($R_9$ = 2-Cl, $R_{10}$ = H) |
| 18 | $CH_3$ | $CH(CH_3)_2$ | Q-2 ($R_9$ = H, $R_{10}$ = 6-Cl) |

| CMPD | $R_7$ | $R_8$ | Q |
|---|---|---|---|
| 19 | $CH_3$ | $CH(CH_3)_2$ | Q-3 ($R_9$ = H, $R_{10}$ = 6-Cl) |
| 20 | $CH_3$ | $CH(CH_3)_2$ | Q-3 ($R_9$ = 2-F, $R_{10}$ = H) |
| 21 | $CH_3$ | $CH(CH_3)_2$ | Q-4 ($R_9$ = 2-F, $R_{10}$ = H) |

## BIOLOGICAL TABLE 2

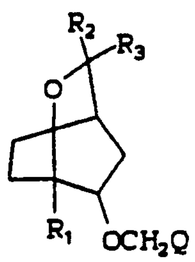

| CMPD | $R_1$ | $R_2$ | $R_3$ | Q |
|---|---|---|---|---|
| 22 | $CH_3$ | $CH_2CH_3$ | $CH_2CH_3$ | Q-1 (X = 2-Cl, Y = H) |
| 23 | $CH_3$ | $CH_2CH_3$ | $CH_2CH_3$ | Q-1 (X = 2-F, Y = 6-F) |
| 24 | $CH_3$ | $CH_2CH_3$ | $CH_2CH_3$ | Q-1 (X = 2-$CH_3$, Y = H) |
| 25 | $CH_3$ | $CH_2CH_3$ | $CH_2CH_3$ | Q-1 (X = 2-Cl, Y = 6-F) |
| 26 | $CH_3$ | $-(CH_2)_4-$ | | Q-1 (X = H, Y = H) |

## BIOLOGICAL TABLE 3

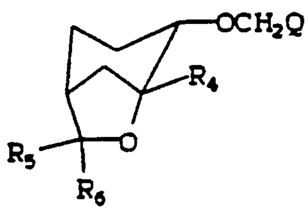

| CMPD | $R_4$ | $R_5$ | $R_6$ | Q |
|---|---|---|---|---|
| 16 | $CH_3$ | $CH_3$ | $CH_3$ | Q-2 ($R_9$ = H, $R_{10}$ = H) |

TEST A

Seeds of barley (Hordeum vulgare), barnyardgrass (Echinochloa crus-galli), cheatgrass (Bromus secalinus), cocklebur (Xanthium pensylvanicum), corn (Zea mays), cotton (Gossypium hirsutum), crabgrass (Digitaria spp.), giant foxtail (Setaria faberi), morningglory (Ipomoea spp.), rice (Oryza sativa), sorghum (Sorghum bicolor), soybean (Glycine max), sugar beet (Beat vulgaris), velvetleaf (Abutilon theophrasti), wheat (Triticum aestivum), and wild oat (Avena fatua) and purple nutsedge (Cyperus rotundus) tubers were planted and treated preemergence with test chemicals dissolved in a non-phytotoxic solvent. At the same time, these crop and weed species were also treated with postemergence applications of test chemicals. Plants ranged in height from two to eighteen cm (two to three leaf stage) for postemergence treatments. Treated plants and controls were maintained in a greenhouse for approximately sixteen days, after which all species were compared to controls and visually evaluated. Plant response ratings, summarized in Table A, are based on a scale of 0 to 10 where 0 is no effect and 10 is complete control. A dash (-) response means no test result.

| Table A | COMPOUND |
|---|---|
| Rate (1000 g/ha) | 5 |
| POSTEMERGENCE | |
| Barley | 8 |
| Barnyardgrass | 9 |
| Cheatgrass | 0 |
| Cocklebur | 2 |
| Corn | 9 |
| Cotton | 0 |
| Crabgrass | 9 |
| Giant foxtail | 9 |
| Morningglory | 0 |
| Nutsedge | 0 |
| Rice | 9 |
| Sorghum | 3 |
| Soybean | 2 |
| Sugar beet | 2 |
| Velvetleaf | 0 |
| Wheat | 8 |
| Wild Oat | 9 |

| Table A | COMPOUND |
|---|---|
| Rate (1000 g/ha) | 5 |
| PREEMERGENCE | |
| Barley | 9 |
| Barnyardgrass | 10 |
| Cheatgrass | 9 |
| Cocklebur | 3 |
| Corn | 10 |
| Cotton | 0 |
| Crabgrass | 10 |
| Giant foxtail | 10 |
| Morningglory | 0 |
| Nutsedge | 9 |
| Rice | 10 |
| Sorghum | 10 |
| Soybean | 9 |
| Sugar beet | 0 |
| Velvetleaf | 8 |
| Wheat | 10 |
| Wild Oat | 9 |

| Table A | COMPOUND | | | | | | | | | | | | | | |
|---|---|---|---|---|---|---|---|---|---|---|---|---|---|---|---|
| Rate (400 g/ha) | 1 | 2 | 3 | 4 | 6 | 8 | 11 | 17 | 18 | 19 | 20 | 21 | 22 | 23 | 24 |
| POSTEMERGENCE | | | | | | | | | | | | | | | |
| Barley | 0 | 0 | 0 | 0 | 4 | 0 | 0 | 0 | 2 | 0 | 4 | 0 | 0 | 0 | 0 |
| Barnyardgrass | 7 | 2 | 1 | 0 | 8 | 2 | 0 | 6 | 8 | 0 | 9 | 0 | 5 | 9 | 2 |
| Cheatgrass | 0 | 0 | 0 | 0 | 2 | 0 | 0 | 0 | 0 | 0 | 0 | 0 | 0 | 0 | 0 |
| Cocklebur | 2 | 1 | 1 | 0 | 1 | 1 | 2 | 2 | 1 | 0 | 1 | 0 | 1 | 1 | 3 |
| Corn | 2 | 0 | 0 | 0 | 7 | 0 | 0 | 0 | 5 | 0 | 6 | 0 | 0 | 0 | 0 |
| Cotton | 2 | 0 | 0 | 0 | 0 | 0 | 0 | 0 | 0 | 0 | 0 | 0 | 8 | 10 | 0 |
| Crabgrass | 7 | 2 | 2 | 0 | 2 | 4 | 2 | 0 | 2 | 0 | 0 | 0 | 9 | 9 | 4 |
| Giant foxtail | 7 | 3 | 0 | 0 | 0 | 0 | 0 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 | 0 |
| Nutsedge | 0 | - | - | 0 | 0 | 0 | 0 | 0 | 5 | 0 | 0 | 0 | - | 0 | 0 |
| Rice | 5 | 2 | 0 | 0 | 0 | 0 | 0 | 0 | 0 | 0 | 0 | 0 | 0 | 0 | 0 |
| Sorghum | 0 | 0 | 0 | 0 | 0 | 0 | 0 | 0 | 0 | 0 | 0 | 0 | 0 | 0 | 0 |
| Soybean | 0 | 2 | 3 | 0 | 1 | 0 | 0 | 1 | 0 | 0 | 0 | 0 | 0 | 1 | 0 |
| Sugar beet | 0 | 0 | 0 | 0 | 0 | 0 | 0 | 0 | 0 | 0 | 0 | 0 | 0 | 0 | 0 |
| Velvetleaf | 8 | 6 | 2 | 0 | 1 | 2 | 1 | 3 | 0 | 0 | 0 | 0 | 6 | 0 | 6 |
| Wheat | 0 | 0 | 0 | 0 | 3 | 0 | 0 | 0 | 0 | 0 | 5 | 0 | 0 | 0 | 0 |
| Wild Oat | 0 | 0 | 0 | 0 | 0 | 0 | 0 | 0 | 0 | 0 | 0 | 0 | 0 | 0 | 0 |

| Table A | COMPOUND | | | | | | | | | | | | | | |
|---|---|---|---|---|---|---|---|---|---|---|---|---|---|---|---|
| Rate (400 g/ha) | 1 | 2 | 3 | 4 | 6 | 8 | 11 | 17 | 18 | 19 | 20 | 21 | 22 | 23 | 24 |
| PREEMERGENCE | | | | | | | | | | | | | | | |
| Barley | 0 | 0 | 0 | 0 | 2 | 0 | 0 | 0 | 1 | 0 | 6 | 0 | 0 | 0 | 0 |
| Barnyardgrass | 9 | 5 | 2 | 0 | 10 | 2 | 10 | 9 | 10 | 7 | 10 | 5 | 10 | 10 | 10 |
| Cheatgrass | 5 | 0 | 0 | 0 | 2 | 0 | 0 | 8 | 6 | 0 | 8 | 0 | 0 | 9 | 5 |
| Cocklebur | 4 | 0 | 0 | 0 | - | 0 | 0 | 0 | 0 | 0 | 0 | 0 | 0 | 0 | 0 |
| Corn | 2 | 0 | 0 | 0 | 5 | 0 | 0 | 5 | 8 | 2 | 7 | 0 | 3 | 8 | 3 |
| Cotton | 2 | 0 | 0 | 0 | 0 | 0 | 0 | 0 | 0 | 0 | 0 | 0 | 0 | 0 | 0 |
| Crabgrass | 9 | 9 | 8 | 8 | 8 | 7 | 8 | 9 | 10 | 2 | 8 | 0 | 9 | 10 | 8 |
| Giant foxtail | 9 | 9 | 9 | 8 | 9 | 8 | 9 | 9 | 10 | 7 | 10 | 8 | 9 | 10 | 9 |
| Morningglory | 1 | 0 | 0 | 0 | 0 | 0 | 0 | 0 | 0 | 0 | 0 | 0 | 0 | 0 | 0 |
| Nutsedge | 0 | 0 | 0 | 10 | 0 | 0 | 0 | 0 | 0 | 0 | 10 | 0 | 7 | 10 | 0 |
| Rice | 0 | 0 | 0 | 0 | 0 | 0 | 0 | 0 | 0 | 0 | 7 | 0 | 0 | 10 | 0 |
| Sorghum | 0 | 0 | 0 | 0 | 8 | 0 | 0 | 3 | 7 | 0 | 5 | 0 | 0 | 6 | 0 |
| Soybean | 0 | 0 | 0 | 0 | 0 | 0 | 0 | 4 | 0 | 0 | 2 | 0 | 0 | 0 | 0 |
| Sugar beet | 0 | 0 | 0 | 0 | 0 | 0 | 0 | 0 | 0 | 0 | 0 | 0 | 0 | 0 | 4 |
| Velvetleaf | 5 | 5 | 2 | 0 | 6 | 2 | 2 | 7 | 7 | 0 | 6 | 3 | 0 | 5 | 0 |
| Wheat | 3 | 0 | 0 | 0 | 5 | 0 | 0 | 5 | 3 | 0 | 7 | 0 | 0 | 0 | 0 |
| Wild Oat | 0 | 0 | 0 | 0 | 0 | 3 | 0 | 0 | 6 | 0 | 7 | 0 | 0 | 0 | 2 |

| Table A | COMPOUND | | | | | | | | |
|---|---|---|---|---|---|---|---|---|---|
| Rate (200 g/ha) | 7 | 9 | 10 | 12 | 13 | 14 | 15 | 16 | 26 |
| POSTEMERGENCE | | | | | | | | | |
| Barley | 0 | 0 | 0 | 0 | 0 | 0 | 9 | 0 | 0 |
| Barnyardgrass | 5 | 0 | 2 | 0 | 0 | 0 | 9 | 1 | 0 |
| Cheatgrass | 0 | 0 | 0 | 0 | 0 | 0 | 8 | 0 | 0 |
| Cocklebur | - | 1 | 0 | 1 | 1 | 1 | 1 | 1 | 1 |
| Corn | 0 | 0 | 0 | 0 | 0 | 0 | 9 | 0 | 0 |
| Cotton | 0 | 0 | 0 | 0 | 0 | 0 | 2 | 0 | 0 |
| Crabgrass | 3 | 3 | 0 | 0 | 0 | 0 | 5 | 0 | 2 |
| Giant foxtail | 2 | 0 | 0 | 2 | 0 | 0 | 9 | 0 | 0 |
| Morningglory | 0 | 0 | 0 | 0 | 0 | 0 | 0 | 0 | 0 |
| Nutsedge | 0 | 0 | 0 | 0 | 0 | 0 | 0 | 0 | 0 |
| Rice | 0 | 0 | 0 | 3 | 0 | 0 | 9 | 0 | 0 |
| Sorghum | 0 | 0 | 0 | 0 | 0 | 0 | 1 | 0 | 0 |
| Soybean | 0 | 0 | 0 | 2 | 0 | 0 | 2 | 0 | 0 |
| Sugar beet | 0 | 0 | 0 | 1 | 0 | 0 | 0 | 0 | 0 |
| Velvetleaf | 0 | 0 | 1 | 3 | 0 | 0 | 2 | 2 | 0 |
| Wheat | 0 | 0 | 0 | 0 | 0 | 0 | 7 | 0 | 0 |
| Wild Oat | 0 | 0 | 0 | 0 | 0 | 0 | 4 | 0 | 0 |

| Table A | COMPOUND | | | | | | | | |
|---|---|---|---|---|---|---|---|---|---|
| Rate (200 g/ha) | 7 | 9 | 10 | 12 | 13 | 14 | 15 | 16 | 26 |
| PREEMERGENCE | | | | | | | | | |
| Barley | 0 | 2 | 0 | 8 | 0 | 0 | 8 | 0 | 0 |
| Barnyardgrass | 8 | 7 | 9 | 8 | 9 | 0 | 10 | 7 | 8 |
| Cheatgrass | 7 | 3 | 2 | 2 | 5 | 0 | 10 | 4 | 2 |
| Cocklebur | 3 | 0 | 0 | - | 0 | - | 2 | 0 | 0 |
| Corn | 0 | 0 | 0 | 7 | 0 | 0 | 8 | 0 | 0 |
| Cotton | 0 | 0 | 0 | 0 | 0 | 0 | 0 | 0 | 0 |
| Crabgrass | 5 | 6 | 9 | 9 | 9 | 4 | 10 | 9 | 0 |
| Giant foxtail | 5 | 6 | 7 | 8 | 10 | 0 | 10 | 9 | 5 |
| Morningglory | 0 | 0 | 0 | 0 | 0 | - | 1 | 0 | 0 |
| Nutsedge | 0 | - | 0 | 0 | 0 | 0 | 4 | 0 | 0 |
| Rice | 0 | 0 | 0 | 5 | 0 | 0 | 9 | 0 | 0 |
| Sorghum | 0 | 0 | 0 | 2 | 7 | 0 | 7 | 0 | 0 |
| Soybean | 0 | 0 | 0 | 0 | 2 | 0 | 7 | 0 | 0 |
| Sugar beet | 0 | 0 | 0 | 0 | 0 | 0 | 1 | 0 | 0 |
| Velvetleaf | 5 | 3 | 2 | 3 | 1 | - | 5 | 1 | 0 |
| Wheat | 0 | 0 | 0 | 3 | 2 | 0 | 7 | 0 | 0 |
| Wild Oat | 0 | 0 | 0 | 0 | 5 | 0 | 6 | 0 | 0 |

| Table A | COMPOUND | | | | | | | | | | | | | | | | | | |
|---|---|---|---|---|---|---|---|---|---|---|---|---|---|---|---|---|---|---|---|
| Rate (100 g/ha) | 1 | 2 | 3 | 4 | 5 | 6 | 7 | 8 | 10 | 11 | 15 | 17 | 18 | 19 | 20 | 21 | 22 | 23 | 24 |
| POSTEMERGENCE | | | | | | | | | | | | | | | | | | | |
| Barley | 0 | 0 | 0 | 0 | 0 | 0 | 0 | 0 | 0 | 0 | 6 | 0 | 0 | 0 | 0 | 0 | 0 | 0 | 0 |
| Barnyardgrass | 3 | 0 | 0 | 0 | 0 | 0 | 0 | 0 | 0 | 0 | 6 | 0 | 0 | 0 | 2 | 0 | 0 | 0 | 0 |
| Cheatgrass | 0 | 0 | 0 | 0 | 0 | 0 | 0 | 0 | 0 | 0 | 0 | 0 | 0 | 0 | 0 | 0 | 0 | 0 | 0 |
| Cocklebur | 0 | 0 | 0 | 0 | 0 | 0 | 0 | 0 | 0 | 1 | - | 0 | 1 | 0 | 0 | 0 | 0 | 1 | 0 |
| Corn | 0 | 0 | 0 | 0 | 0 | 0 | 0 | 0 | 0 | 0 | 7 | 0 | 0 | 0 | 1 | 0 | 0 | 0 | 0 |
| Cotton | 0 | 0 | 0 | 0 | 0 | 0 | 0 | 0 | 0 | 0 | 0 | 0 | 0 | 0 | 0 | 0 | 0 | 0 | 0 |
| Crabgrass | 0 | 0 | 0 | 0 | 0 | 0 | 0 | 0 | 0 | 0 | 3 | 0 | 0 | 0 | 0 | 0 | 4 | 0 | 2 |
| Giant foxtail | 0 | 0 | 0 | 0 | 0 | 0 | 0 | 0 | 0 | 0 | 9 | 0 | 0 | 0 | 0 | 0 | 0 | 0 | 0 |
| Morningglory | 0 | 0 | 0 | 0 | 0 | 0 | 0 | 0 | 0 | 0 | 0 | 0 | 0 | 0 | 0 | 0 | 0 | 0 | 0 |
| Nutsedge | 0 | 0 | 0 | 0 | 0 | 0 | 0 | 0 | 0 | 0 | 0 | 0 | 0 | 0 | 0 | 0 | 0 | 0 | 0 |
| Rice | 0 | 0 | 0 | 0 | 0 | 0 | 0 | 0 | 0 | 0 | 6 | 0 | 0 | 0 | 0 | 0 | 0 | 0 | 0 |
| Sorghum | 0 | 0 | 0 | 0 | 0 | 0 | 0 | 0 | 0 | 0 | 0 | 0 | 0 | 0 | 0 | 0 | 0 | 0 | 0 |
| Soybean | 0 | 0 | 0 | 0 | 0 | 0 | 0 | 0 | 0 | 0 | 1 | 0 | 0 | 0 | 0 | 0 | 0 | 0 | 0 |
| Sugar beet | 0 | 0 | 0 | 0 | 0 | 0 | 0 | 0 | 0 | 0 | 0 | 0 | 0 | 0 | 0 | 0 | 0 | 0 | 0 |
| Velvetleaf | 0 | 0 | 0 | 0 | 0 | 0 | 0 | 0 | 0 | 0 | 1 | 0 | 0 | 0 | 0 | 0 | 0 | 0 | 5 |
| Wheat | 0 | 0 | 0 | 0 | 0 | 0 | 0 | 0 | 0 | 0 | - | 0 | 0 | 0 | 0 | 0 | 0 | 0 | 0 |
| Wild Oat | 0 | 0 | 0 | 0 | 0 | 0 | 0 | 0 | 0 | 0 | 3 | 0 | 0 | 0 | 0 | 0 | 0 | 0 | 0 |

| Table A | COMPOUND | | | | | | | | | | | | | | | | | | |
|---|---|---|---|---|---|---|---|---|---|---|---|---|---|---|---|---|---|---|---|
| Rate (100 g/ha) | 1 | 2 | 3 | 4 | 5 | 6 | 7 | 8 | 10 | 11 | 15 | 17 | 18 | 19 | 20 | 21 | 22 | 23 | 24 |
| PREEMERGENCE | | | | | | | | | | | | | | | | | | | |
| Barley | 0 | 0 | 0 | 0 | 3 | 0 | 0 | 0 | 0 | 0 | 7 | 0 | 0 | 0 | 0 | 0 | 0 | 0 | 0 |
| Barnyardgrass | 8 | 0 | 0 | 0 | 7 | 10 | 0 | 0 | 4 | 1 | 10 | 6 | 9 | 0 | 7 | 0 | 9 | 9 | 9 |
| Cheatgrass | 0 | 0 | 0 | 0 | 0 | 0 | 2 | 0 | 0 | 8 | 7 | 0 | 0 | 0 | 0 | 0 | 0 | 3 | 0 |
| Cocklebur | 0 | 1 | 0 | 0 | 2 | 0 | 0 | 0 | 0 | 0 | 2 | 0 | 0 | 0 | 0 | 0 | 0 | 0 | 0 |
| Corn | 0 | 0 | 0 | 0 | 2 | 3 | 0 | 0 | 0 | 0 | 6 | 0 | 0 | 0 | 2 | 0 | 0 | 0 | 0 |
| Cotton | 0 | 0 | 0 | 0 | 0 | 0 | 0 | 0 | 0 | 0 | 0 | 0 | 0 | 0 | 0 | 0 | 0 | 0 | 0 |
| Crabgrass | 8 | 5 | 0 | 2 | 8 | 7 | 2 | 2 | 7 | 0 | 10 | 3 | 9 | 0 | 7 | 0 | 7 | 8 | 7 |
| Giant foxtail | 9 | 0 | 7 | 5 | 7 | 8 | 0 | 3 | 3 | 2 | 10 | 8 | 9 | 0 | 9 | 0 | 9 | 9 | 7 |
| Morningglory | 0 | 0 | 0 | 0 | 0 | 0 | 0 | 0 | 0 | 0 | 0 | 0 | 0 | 0 | 0 | 0 | 0 | 0 | 0 |
| Nutsedge | - | 7 | 0 | 0 | 0 | 0 | 0 | 0 | 0 | 0 | - | 0 | 0 | 0 | 0 | 0 | 0 | 0 | 0 |
| Rice | 0 | 0 | 0 | 0 | 0 | 0 | 0 | 0 | 0 | 0 | 7 | 0 | 0 | 0 | 0 | 0 | 0 | 0 | 0 |
| Sorghum | 0 | 0 | 0 | 0 | 0 | 0 | 0 | 0 | 0 | 0 | 6 | 0 | 0 | 0 | 0 | 0 | 0 | 0 | 0 |
| Soybean | 0 | 0 | 0 | 0 | 0 | 0 | 0 | 0 | 0 | 0 | 1 | 0 | 0 | 0 | 0 | 0 | 0 | 0 | 0 |
| Sugar beet | 0 | 0 | 0 | 0 | 0 | 0 | 0 | 0 | 0 | 0 | 0 | 0 | 0 | 0 | 0 | 0 | 0 | 0 | 0 |
| Velvetleaf | 1 | 0 | 0 | 0 | 1 | 1 | 0 | 0 | 0 | - | 3 | 3 | 1 | 0 | 1 | 0 | 0 | 1 | 0 |
| Wheat | 0 | 0 | 0 | 0 | 3 | 0 | 0 | 0 | 0 | 0 | 7 | 0 | 0 | 0 | 2 | 0 | 0 | 0 | 0 |
| Wild Oat | 0 | 0 | 0 | 0 | 0 | 0 | 0 | 0 | 0 | 0 | 4 | 0 | 0 | 0 | 3 | 0 | 0 | 0 | 0 |

| Table A | COMPOUND | | | | | | | | |
|---|---|---|---|---|---|---|---|---|---|
| Rate (50 g/ha) | 7 | 9 | 10 | 12 | 13 | 14 | 15 | 16 | 26 |
| POSTEMERGENCE | | | | | | | | | |
| Barley | 0 | 0 | 0 | 0 | 0 | 0 | 0 | 0 | 0 |
| Barnyardgrass | 0 | 0 | 0 | 0 | 0 | 0 | 0 | 0 | 0 |
| Cheatgrass | 0 | 0 | 0 | 0 | 0 | 0 | 0 | 0 | 0 |
| Cocklebur | 0 | 0 | 0 | 0 | 0 | 0 | 1 | 0 | 0 |
| Corn | 0 | 0 | 0 | 0 | 0 | 0 | 2 | 0 | 0 |
| Cotton | 0 | 0 | 0 | 0 | 0 | 0 | 0 | 0 | 0 |
| Crabgrass | 0 | 0 | 0 | 0 | 0 | 0 | 1 | 0 | 0 |
| Giant foxtail | 0 | 0 | 0 | 0 | 0 | 0 | 2 | 0 | 0 |
| Morningglory | 0 | 0 | 0 | 0 | 0 | 0 | 0 | 0 | 0 |
| Nutsedge | 0 | 0 | - | - | 0 | 0 | 0 | 0 | 0 |
| Rice | 0 | 0 | 0 | 0 | 0 | 0 | 0 | 0 | 0 |
| Sorghum | 0 | 0 | 0 | 0 | 0 | 0 | 0 | 0 | 0 |
| Soybean | 0 | 0 | 0 | 0 | 0 | 0 | 0 | 0 | 0 |
| Sugar beet | 0 | 0 | 0 | 0 | 0 | 0 | 0 | 0 | 0 |
| Velvetleaf | 0 | 0 | 0 | 3 | 0 | 0 | 0 | 0 | 0 |
| Wheat | 0 | 0 | 0 | 0 | 0 | 0 | 0 | 0 | 0 |
| Wild Oat | 0 | 0 | 0 | 0 | 0 | 0 | 0 | 0 | 0 |

| Table A | COMPOUND | | | | | | | | |
|---|---|---|---|---|---|---|---|---|---|
| Rate (50 g/ha) | 7 | 9 | 10 | 12 | 13 | 14 | 15 | 16 | 26 |
| PREEMERGENCE | | | | | | | | | |
| Barley | 0 | 0 | 0 | 0 | 0 | 0 | 3 | 0 | 0 |
| Barnyardgrass | 0 | 0 | 2 | 8 | 9 | 0 | 10 | 0 | 0 |
| Cheatgrass | 0 | 0 | 0 | 0 | 0 | 0 | 4 | 0 | 0 |
| Cocklebur | 0 | 0 | 0 | 0 | 0 | - | 0 | - | 0 |
| Corn | 0 | 0 | 0 | 0 | 0 | 0 | 3 | 0 | 0 |
| Cotton | 0 | 0 | 0 | 0 | 0 | 0 | 0 | 0 | 0 |
| Crabgrass | 0 | 5 | 0 | 3 | 9 | 3 | 10 | 1 | 0 |
| Giant foxtail | 0 | 2 | 2 | 8 | 7 | 0 | 10 | 3 | 0 |
| Morningglory | 0 | 0 | 0 | 0 | 0 | 0 | 0 | 0 | 0 |
| Nutsedge | 0 | 0 | - | 0 | 0 | 0 | 0 | 0 | 0 |
| Rice | 0 | 0 | 0 | 1 | 0 | 0 | 0 | 0 | 0 |
| Sorghum | 0 | 0 | 0 | 2 | 0 | 0 | 2 | 0 | 0 |
| Soybean | 0 | 0 | 0 | 4 | 0 | 0 | 0 | 0 | 0 |
| Sugar beet | 0 | 0 | 0 | 0 | 0 | 0 | 0 | 0 | 0 |
| Velvetleaf | 0 | 0 | 0 | 0 | 0 | 0 | 1 | 0 | 0 |
| Wheat | 0 | 0 | 0 | 0 | 0 | 0 | 2 | 0 | 0 |
| Wild Oat | 0 | 0 | 0 | 0 | 0 | 0 | 2 | 0 | 0 |

| Table A | COMPOUND |
|---|---|
| Rate (20 g/ha) | 5 |
| POSTEMERGENCE | |
| Barley | 0 |
| Barnyardgrass | 0 |
| Cheatgrass | 0 |
| Cocklebur | 0 |
| Corn | 0 |
| Cotton | 0 |
| Crabgrass | 0 |
| Giant foxtail | 0 |
| Morningglory | 0 |
| Nutsedge | 0 |
| Rice | 0 |
| Sorghum | 0 |
| Soybean | 0 |
| Sugar beet | 0 |
| Velvetleaf | 0 |
| Wheat | 0 |
| Wild Oat | 0 |

| Table A | COMPOUND |
|---|---|
| Rate (20 g/ha) | 5 |
| PREEMERGENCE | |
| Barley | 0 |
| Barnyardgrass | 0 |
| Cheatgrass | 0 |
| Cocklebur | 0 |
| Corn | 0 |
| Cotton | 0 |
| Crabgrass | 0 |
| Giant foxtail | 0 |
| Morningglory | 0 |
| Nutsedge | 0 |
| Rice | 0 |
| Sorghum | 0 |
| Soybean | 0 |
| Sugar beet | 0 |
| Velvetleaf | 0 |
| Wheat | 0 |
| Wild Oat | 0 |

## TEST B

Seeds of barley (Hordeum vulgare), barnyardgrass (Echinochloa crus-galli), blackgrass (Alopecurus myosuroides), chickweed (Stellaria media), cocklebur (Xanthium pensylvanicum), corn (Zea mays), cotton (Gossypium hirsutum), crabgrass (Digitaria spp.), downy brome (Bromus tectorum), giant foxtail (Setaria faberi), green foxtail (Setaria viridis), jimsonweed (Datura stramonium), johnsongrass (Sorghum halepense), lambsquarters (Chenopodium album), morningglory (Ipomoea spp.), rape (Brassica napus), rice (Oryza sativa), sicklepod (Cassia obtusifolia), soybean (Glycine max), sugar beet (Beta vulgaris), teaweed (Sida spinosa), velvetleaf (Abutilon theophrasti), wheat (Triticum aestivum), wild buckwheat (Polygonum convolvulus), and wild oat (Avena fatua) and purple nutsedge (Cyperus rotundus) tubers were planted and treated preemergence with test chemicals dissolved in a non-phytotoxic solvent. At the same time, these crop and weed species were also treated with postemergence applications of test chemicals. Plants ranged in height from two to eighteen cm (two to three leaf stage) for postemergence treatments. Treated plants and controls wer maintained in a greenhouse for approximately 24 days, after which all species were compared to controls and visually evaluated. Plant response ratings, summarized in Table B, are reported on a 0 to 10 scale where 0 is no effect and 10 is complete control. A dash (-) response means no test result.

| Table B | COMPOUND |
|---|---|
| Rate (500 g/ha) | 6 |
| PREEMERGENCE | |
| Barley | 4 |
| Barnyardgrass | 10 |
| Blackgrass | 7 |
| Chickweed | 0 |
| Cocklebur | 3 |
| Corn | 4 |
| Cotton | 2 |
| Crabgrass | 10 |
| Downy brome | 3 |
| Giant foxtail | 10 |
| Green foxtail | 10 |
| Jimsonweed | 0 |
| Johnsongrass | 7 |
| Morningglory | 0 |
| Nutsedge | 0 |
| Rape | 2 |
| Sicklepod | 0 |
| Soybean | 0 |
| Sugar beet | 0 |
| Teaweed | 3 |
| Velvetleaf | 3 |
| Wheat | 3 |
| Wild buckwheat | 0 |
| Wild oat | 5 |

| Table B | COMPOUND | | |
|---|---|---|---|
| Rate (250 g/ha) | 5 | 13 | 15 |
| POSTEMERGENCE | | | |
| Barley | 0 | 4 | 9 |
| Barnyardgrass | 0 | 10 | 10 |
| Blackgrass | 0 | 2 | 10 |
| Chickweed | 5 | 0 | 4 |
| Cocklebur | - | 3 | 0 |
| Corn | 0 | 8 | 5 |
| Cotton | 0 | 2 | 0 |
| Crabgrass | 7 | 8 | 6 |
| Downy brome | 0 | 2 | 9 |
| Giant foxtail | 0 | 6 | 10 |
| Green foxtail | 0 | 6 | 5 |
| Jimsonweed | 6 | 3 | 0 |
| Johnsongrass | 0 | 6 | 8 |
| Lambsquarters | 3 | 10 | - |
| Morningglory | 6 | 3 | 0 |
| Nutsedge | 4 | 0 | 5 |
| Rape | 0 | 8 | 0 |
| Rice | 0 | 3 | 2 |
| Sicklepod | 0 | 0 | 0 |
| Soybean | 0 | 3 | 0 |
| Sugar beet | 0 | 5 | 0 |
| Teaweed | 4 | 3 | 0 |
| Velvetleaf | 0 | 4 | 0 |
| Wheat | 0 | 0 | 9 |
| Wild buckwheat | 0 | 5 | 6 |
| Wild oat | 0 | 0 | 9 |

| Table B | COMPOUND | | | |
|---|---|---|---|---|
| Rate (250 g/ha) | 5 | 6 | 13 | 15 |
| PREEMERGENCE | | | | |
| Barley | 0 | 3 | 5 | 9 |
| Barnyardgrass | 10 | 10 | 10 | 10 |
| Blackgrass | 0 | 5 | 10 | 7 |
| Chickweed | 10 | 0 | 9 | 7 |
| Cocklebur | 8 | 2 | 0 | 7 |
| Corn | 3 | 3 | 10 | 6 |
| Cotton | 3 | 0 | 0 | 6 |
| Crabgrass | 10 | 9 | 10 | 10 |
| Downy brome | 0 | 2 | 4 | 10 |
| Giant foxtail | 6 | 9 | 10 | 10 |
| Green foxtail | 6 | 9 | 10 | 10 |
| Jimsonweed | 0 | 0 | 3 | 4 |
| Johnsongrass | 5 | 5 | 0 | 9 |
| Lambsquarters | 8 | - | 9 | - |
| Morningglory | 0 | 0 | 0 | 6 |
| Nutsedge | 0 | 0 | 4 | 7 |
| Rape | - | 0 | 0 | 0 |
| Rice | 5 | - | 10 | 7 |
| Sicklepod | 4 | 0 | 0 | 5 |
| Soybean | 0 | 0 | 3 | 3 |
| Sugar beet | - | 0 | 0 | 4 |
| Teaweed | 8 | 3 | 6 | 5 |
| Velvetleaf | 5 | 3 | 8 | 10 |
| Wheat | 0 | 2 | 5 | 7 |
| Wild buckwheat | - | 0 | 3 | 7 |
| Wild oat | - | 4 | 10 | 8 |

| Table B | COMPOUND | | |
|---|---|---|---|
| Rate (125 g/ha) | 5 | 13 | 15 |
| POSTEMERGENCE | | | |
| Barley | 0 | 2 | 0 |
| Barnyardgrass | 0 | 7 | 9 |
| Blackgrass | 0 | 2 | 8 |
| Chickweed | 5 | 0 | 0 |
| Cocklebur | - | 2 | 0 |
| Corn | 0 | 3 | 3 |
| Cotton | 0 | 2 | 0 |
| Crabgrass | 0 | 3 | 3 |
| Downy brome | 0 | 0 | 0 |
| Giant foxtail | 0 | 5 | 6 |
| Green foxtail | 0 | 4 | 1 |
| Jimsonweed | 0 | 3 | 0 |
| Johnsongrass | 0 | 3 | 5 |
| Lambsquarters | 0 | 3 | - |
| Morningglory | 0 | 0 | 0 |
| Nutsedge | 4 | 0 | 4 |
| Rape | 0 | 0 | 0 |
| Rice | 0 | 0 | 0 |
| Sicklepod | 0 | 0 | 0 |
| Soybean | 0 | 3 | 0 |
| Sugar beet | 0 | 2 | 0 |
| Teaweed | 0 | 3 | 0 |
| Velvetleaf | 0 | 2 | - |
| Wheat | 0 | 0 | 2 |
| Wild buckwheat | 0 | 0 | 0 |
| Wild oat | 0 | 0 | 2 |

| Table B | COMPOUND | | |
|---|---|---|---|
| Rate (125 g/ha) | 5 | 13 | 15 |
| PREEMERGENCE | | | |
| Barley | 0 | 5 | 6 |
| Barnyardgrass | 10 | 10 | 10 |
| Blackgrass | 0 | 10 | 5 |
| Chickweed | 7 | 8 | 5 |
| Cocklebur | 3 | 0 | 3 |
| Corn | 2 | 0 | 6 |
| Cotton | 0 | 0 | 4 |
| Crabgrass | 10 | 10 | 10 |
| Downy brome | 0 | 4 | 10 |
| Giant foxtail | 5 | 10 | 10 |
| Green foxtail | 0 | 10 | 10 |
| Jimsonweed | 0 | 0 | 3 |
| Johnsongrass | 0 | 0 | 7 |
| Lambsquarters | 6 | - | - |
| Morningglory | 0 | 0 | 5 |
| Nutsedge | - | - | 5 |
| Rape | - | 0 | 0 |
| Rice | 3 | - | 4 |
| Sicklepod | 0 | - | 3 |
| Soybean | 0 | 2 | 2 |
| Sugar beet | - | - | 3 |
| Teaweed | 3 | 4 | 4 |
| Velvetleaf | 3 | - | 10 |
| Wheat | 0 | 3 | 6 |
| Wild buckwheat | - | 3 | 3 |
| Wild oat | - | 8 | 6 |

| Table B | COMPOUND |
|---|---|
| Rate (62 g/ha) | 13 |
| POSTEMERGENCE | |
| Barley | 0 |
| Barnyardgrass | 4 |
| Blackgrass | 0 |
| Chickweed | 0 |
| Cocklebur | 0 |
| Corn | 0 |
| Cotton | 0 |
| Crabgrass | 0 |
| Downy brome | 0 |
| Giant foxtail | 0 |
| Green foxtail | 0 |
| Jimsonweed | 0 |
| Johnsongrass | 0 |
| Lambsquarters | 3 |
| Morningglory | 0 |
| Nutsedge | 0 |
| Rape | 0 |
| Rice | 0 |
| Sicklepod | 0 |
| Soybean | 0 |
| Sugar beet | 0 |
| Teaweed | 0 |
| Velvetleaf | 0 |
| Wheat | 0 |
| Wild buckwheat | 0 |
| Wild oat | 0 |

| Table B | COMPOUND | | | |
|---|---|---|---|---|
| Rate (62 g/ha) | 5 | 6 | 13 | 15 |
| PREEMERGENCE | | | | |
| Barley | 0 | 2 | 5 | 4 |
| Barnyardgrass | 10 | 10 | 10 | 10 |
| Blackgrass | 0 | 2 | 8 | 3 |
| Chickweed | 7 | 0 | 3 | 3 |
| Cocklebur | - | 0 | 0 | - |
| Corn | 2 | 2 | 0 | 2 |
| Cotton | 0 | 0 | 0 | 4 |
| Crabgrass | 8 | 7 | 10 | 10 |
| Downy brome | 0 | 0 | 2 | 5 |
| Giant foxtail | 2 | 7 | 10 | 10 |
| Green foxtail | 0 | 6 | 10 | 10 |
| Jimsonweed | 0 | 0 | 0 | 3 |
| Johnsongrass | 0 | 3 | 0 | 6 |
| Lambsquarters | 5 | - | 0 | - |
| Morningglory | 0 | 0 | 0 | 5 |
| Nutsedge | - | 0 | 0 | 4 |
| Rape | 5 | 0 | 0 | 0 |
| Rice | 2 | 2 | 9 | 3 |
| Sicklepod | 0 | 0 | 0 | 0 |
| Soybean | 0 | 0 | 0 | 2 |
| Sugar beet | - | 0 | 0 | 0 |
| Teaweed | 0 | 0 | 0 | 3 |
| Velvetleaf | 0 | 0 | 0 | 5 |
| Wheat | 0 | 0 | 0 | 6 |
| Wild buckwheat | - | 0 | 0 | 0 |
| Wild oat | - | 2 | 0 | 5 |

| Table B | COMPOUND |
|---|---|
| Rate (31 g/ha) | 13 |
| POSTEMERGENCE | |
| Barley | 0 |
| Barnyardgrass | 0 |
| Blackgrass | 0 |
| Chickweed | 0 |
| Cocklebur | 0 |
| Corn | 0 |
| Cotton | 0 |
| Crabgrass | 0 |
| Downy brome | 0 |
| Giant foxtail | 0 |
| Green foxtail | 0 |
| Johnsongrass | 0 |
| Lambsquarters | 2 |
| Morningglory | 0 |
| Nutsedge | 0 |
| Rape | 0 |
| Rice | 0 |
| Sicklepod | 0 |
| Soybean | 0 |
| Sugar beet | 0 |
| Teaweed | 0 |
| Velvetleaf | 0 |
| Wheat | 0 |
| Wild buckwheat | 0 |
| Wild oat | 0 |

| Table B | COMPOUND | | | |
|---|---|---|---|---|
| Rate (31 g/ha) | 5 | 6 | 13 | 15 |
| PREEMERGENCE | | | | |
| Barley | 0 | 0 | 0 | 2 |
| Barnyardgrass | 4 | 8 | 8 | 10 |
| Blackgrass | - | 0 | 6 | 0 |
| Chickweed | 3 | 0 | 0 | 0 |
| Cocklebur | - | 0 | 0 | 3 |
| Corn | 0 | - | 0 | 0 |
| Cotton | 0 | 0 | 0 | 3 |
| Crabgrass | 5 | 3 | 9 | 9 |
| Downy brome | 0 | 0 | 0 | 4 |
| Giant foxtail | 0 | 4 | 7 | 7 |
| Green foxtail | 0 | 3 | 4 | 6 |
| Jimsonweed | 0 | 0 | 0 | 3 |
| Johnsongrass | 0 | 0 | 0 | 5 |
| Lambsquarters | 0 | - | - | - |
| Morningglory | 0 | 0 | 0 | 4 |
| Nutsedge | 0 | 0 | 0 | 3 |
| Rape | 5 | 0 | 0 | 0 |
| Rice | 0 | 0 | 2 | 2 |
| Sicklepod | 0 | 0 | 0 | 0 |
| Soybean | 0 | 0 | 0 | 0 |
| Sugar beet | - | 0 | 0 | 0 |
| Teaweed | 0 | 0 | 0 | 3 |
| Velvetleaf | 0 | 0 | 0 | 3 |
| Wheat | 0 | 0 | 0 | 3 |
| Wild buckwheat | - | 0 | 0 | 0 |
| Wild oat | - | 0 | 0 | 3 |

TEST C

Plastic pots were partially filled with silt loam soil. The soil was then saturated with water. Indica and Japonica rice (Oryza sativa) seedlings at the 2.0 to 2.5 leaf stage, seeds of barnyardgrass (Echinochloa crus-galli), bulrush (Scirpus mucronatus), duck salad (Heteranthera limosa), and umbrella sedge (Cyperus difformis), and tubers of arrowhead (Sagittaria spp.) and waterchestnut (Eleocharis spp.) were planted into this soil. Several days after planting, water levels were raised to 3 cm above the soil surface and maintained at this level throughout the test. Chemical treatments were formulated in a non-phytotoxic solvent and applied directly to the paddy water. Treated plants and controls were maintained in a greenhouse for approximately 21 days, after which all species were compared to controls and visually evaluated. Plant

response ratings, summarized in Table C, are reported on a 0 to 10 scale where 0 is no effect and 10 is complete control. A dash (-) response means no test result.

| Table C | COMPOUND | | | |
|---|---|---|---|---|
| Rate (500 g/ha) | 5 | 6 | 7 | 13 |
| PADDY | | | | |
| Arrowhead | 0 | - | 0 | 0 |
| Barnyardgrass | 10 | 10 | 10 | 10 |
| Bulrush | 10 | 10 | 10 | 8 |
| Duck salad | - | 10 | 10 | 10 |
| Indica rice | 8 | 9 | 2 | 8 |
| Japonica rice | 9 | 7 | 3 | 9 |
| Umbrella sedge | 10 | 10 | 10 | 10 |
| Waterchestnut | 9 | 7 | 0 | 7 |

| Table C | COMPOUND | | | |
|---|---|---|---|---|
| Rate (250 g/ha) | 5 | 6 | 7 | 13 |
| PADDY | | | | |
| Arrowhead | 0 | - | 0 | 0 |
| Barnyardgrass | 10 | 9 | 10 | 10 |
| Bulrush | 9 | 9 | 9 | 8 |
| Duck salad | - | 10 | 10 | 10 |
| Indica rice | 7 | 6 | 0 | 2 |
| Japonica rice | 8 | 4 | 0 | 7 |
| Umbrella sedge | 10 | 10 | 10 | 10 |
| Waterchestnut | 8 | 4 | 0 | 7 |

| Table C | COMPOUND | | | |
|---|---|---|---|---|
| Rate (125 g/ha) | 5 | 6 | 7 | 13 |
| PADDY | | | | |
| Arrowhead | 0 | - | 0 | 0 |
| Barnyardgrass | 10 | 10 | 8 | 10 |
| Bulrush | 9 | 0 | 0 | 0 |
| Duck salad | - | 8 | 0 | 8 |
| Indica rice | 3 | 0 | 0 | 0 |
| Japonica rice | 6 | 0 | 0 | 0 |
| Umbrella sedge | 10 | 9 | 0 | 10 |
| Waterchestnut | 8 | 4 | 0 | 0 |

| Table C | COMPOUND | | | |
|---|---|---|---|---|
| Rate (64 g/ha) | 5 | 6 | 7 | 13 |
| PADDY | | | | |
| Arrowhead | 0 | - | 0 | 0 |
| Barnyardgrass | 10 | 7 | 0 | 10 |
| Bulrush | 8 | 0 | 0 | 0 |
| Duck salad | - | 6 | 0 | 7 |
| Indica rice | 3 | 0 | 0 | 0 |
| Japonica rice | 4 | 0 | 0 | 0 |
| Umbrella sedge | 9 | 5 | 0 | 10 |
| Waterchestnut | 7 | 0 | 0 | 0 |

| Table C | COMPOUND | | | |
|---|---|---|---|---|
| Rate (32 g/ha) | 5 | 6 | 7 | 13 |
| PADDY | | | | |
| Arrowhead | 0 | - | 0 | 0 |
| Barnyardgrass | 9 | 0 | 0 | 8 |
| Bulrush | 0 | 0 | 0 | 0 |
| Duck salad | - | 0 | 0 | 0 |
| Indica rice | 2 | 0 | 0 | 0 |
| Japonica rice | 2 | 0 | 0 | 0 |
| Umbrella sedge | 6 | 0 | 0 | 0 |
| Waterchestnut | 0 | 0 | 0 | 0 |

## TEST D

This test illustrates herbicidal activity of several compounds of this invention on barnyardgrass (Echinochloa crus-galli) at two growth stages with crop safety to direct-seeded and transplanted Japonica rice (Oryza sativa).

Japonica rice (var. M202) was grown in metromix in 2-cm wide cavity trays until plants were in the three leaf stage. These seedlings were then transplanted into 10-cm diameter pots filled with silt loam soil previously flooded to a depth of 1 cm of water above the soil surface. After 3 days, the water level was then raised to a depth of 3 cm. Test compounds were applied in a non-phytotoxic solvent directly to the paddy water one day after the water level was raised to 3 cm. This 3-cm water level was maintained until test results were recorded.

For direct seeded rice, Japonica rice (var. M202) was previously germinated on moistened burlap until radicals appeared. Uniformly germinated seeds were selected and placed on the surface of a silt loam soil in 10-cm diameter pots. Sufficient water was added to keep the soil surface muddy without covering the seeds with water. One day prior to treatment, the water level was raised to 3 cm above the soil surface and maintained at this level throughout the experiment. Test chemicals, dissolved in a non-phytotoxic solvent, were applied when rice seedlings were in the 1.5 leaf stage.

Barnyardgrass seeds were mixed uniformly into silt loam soil. A 2-cm depth of this seed-soil mix was placed into 10-cm diameter pots partially filled with silt loam soil. Sufficient water was added to keep the soil muddy without flooding the soil surface. One day prior to treatment, the pots were flooded to a depth of

3 cm and maintained at this water level for the duration of the test. Test chemicals, dissolved in a non-phytotoxic solvent, were applied directly to the paddy water when the barnyardgrass was either in the 1 leaf stage or in the 2 leaf stage. Planting times for all species were scheduled so that test chemicals were applied to all plants at the same time.

Plant response ratings, shown in Table D, were recorded 14 days after application of test chemicals. These ratings are reported on a 0 to 10 scale where 0 is no injury and 10 is plant death. A dash (-) response means no test result.

Table D

| PADDY | COMPOUND | | | |
|---|---|---|---|---|
| | 22 | 23 | 24 | 25 |
| Rate (1000 g/ha) | | | | |
| Barnyardgrass (1 leaf) | 10 | 10 | 10 | 10 |
| Barnyardgrass (2 leaf) | 10 | 10 | 10 | 10 |
| Rice (direct-seeded) | 2 | 6 | 7 | 3 |
| Rice (transplanted) | 2 | 3 | 0 | 0 |
| Rate (500 g/ha) | | | | |
| Barnyardgrass (1 leaf) | 10 | 10 | - | 10 |
| Barnyardgrass (2 leaf) | 10 | 10 | - | 10 |
| Rice (direct-seeded) | 0 | 2 | - | 2 |
| Rice (transplanted) | 0 | 0 | - | 0 |
| Rate (250 g/ha) | | | | |
| Barnyardgrass (1 leaf) | 10 | 10 | 10 | 10 |
| Barnyardgrass (2 leaf) | 10 | 10 | 10 | 9 |
| Rice (direct-seeded) | 0 | 0 | 0 | 0 |
| Rice (transplanted) | 0 | 0 | 0 | 0 |
| Rate (100 g/ha) | | | | |
| Barnyardgrass (1 leaf) | 10 | 10 | 10 | 10 |
| Barnyardgrass (2 leaf) | 9 | 9 | 10 | 8 |
| Rice (direct-seeded) | 0 | 0 | 0 | 0 |
| Rice (transplanted) | 0 | 0 | 0 | 0 |
| Rate (40 g/ha) | | | | |
| Barnyardgrass (1 leaf) | 7 | 9 | 5 | 10 |
| Barnyardgrass (2 leaf) | 4 | 6 | 6 | 4 |
| Rice (direct-seeded) | 0 | 0 | 0 | 0 |
| Rice (transplanted) | 0 | 0 | 0 | 0 |
| Rate (16 g/ha) | | | | |
| Barnyardgrass (1 leaf) | 6 | 8 | 0 | 3 |
| Barnyardgrass (2 leaf) | 0 | 3 | 0 | 0 |
| Rice (direct-seeded) | 0 | 0 | 0 | 0 |
| Rice (transplanted) | 0 | 0 | 0 | 0 |

## SPECTRAL DATA TABLE

| CMPD | Data |
|---|---|
| 4 | Proton NMR ($CDCl_3$, 200 MHz, ppm): 1.0 (m, 6H), 1.4-1.6 (m, 7H), 1.9-2.2 (m, 3H), 3.58 (m, 1H), 4.5 (q, 2H), 7.26 (m, 1H), 7.7 (m, 1H), 8.55 (m, 2H). |
| 5 | Proton NMR ($CDCl_3$, 90 HMz, ppm): 0.98 (d, 6H, J = 7 Hz), 1.49 (s, 3H), 1.3-1.7 (m, 4H), 1.8-2.2 (m, 3H), 3.68 (dd, 1H, J = 2.8, 6.5 Hz), 4.57 (d, 1H, J = 12 Hz), 4.78 (d, 1H, J = 12 Hz), 7.31 (fine m, 1H), 8.75 (d, 1H, J = 1.5 Hz). |
| | Carbon NMR ($CDCl_3$, 100 mHz, ppm): 16.4, 17.9, 18.1, 31.6, 32.5, 33.7, 42.2, 66.6, 83.7, 85.2, 88.4, 114.9, 152.5, 155.3. |
| 8 | Proton NMR ($CDCl_3$, 200 MHz, ppm): 1.0 (d, 6H), 1.5 (m, 8H), 2.0 (m, 2H), 2.5 (s, 3H), 3.6 (dd, 1H), 4.59 (q, 2H), 7.0 (dd, 1H), 7.31 (d, 1H), 7.56 (t, 1H). |
| 13 | Infrared (neat): 2990, 1605, 1580, 1430, 1110, 920 (broad), 790, 710 $cm^{-1}$. |
| 14 | Proton NMR ($CDCl_3$, 200 MHz, ppm): 0.95 (t, 6H), 1.3-2.3 (m, 10H), 2.5 (s, 3H), 3.7 (dd, 1H), 4.5 (q, 2H), 7.0 (d, 1H), 7.4 (d, 1H), 7.55 (t, 1H). |
| | Infrared (neat): 2980, 1590, 1580, 1455, 1100 (broad), 780, 710, $cm^{-1}$. |
| 15 | Proton NMR ($CDCl_3$, 90 MHz, ppm): 0.93 (t, 3H, J = 7.5 Hz), 0.98 (t, 3H, J = 7.5 Hz), 1.3-2.3 (m, 10H), 3.75 (dd, 1H, J = 2.5, 7 Hz), 4.52 (d, 1H, J = 12 Hz), 4.73 (d, 1H, J = 12 Hz), 7.29 (fine m, 1H), 8.77 (d, 1H, J = 2.5 Hz). |

| CMPD | Data |
|---|---|
| 16 | Proton NMR ($CDCl_3$, 200 MHz, ppm): 1.1-1.6 (m, 11H), 1.8 (m, 3H), 1.9-2.2 (m, 2H), 3.2 (dd, 1H), 4.6 (q, 2H), 7.1 (dd, 1H), 7.5-7.7 (m, 2H), 8.45 (dd, 1H). Infrared (neat): 2970, 1590, 1580, 1450, 1430, 1360, 1100, 960, 750, 720 $cm^{-1}$. |

## Claims

1. A method for controlling the growth of undesired vegetation in a paddy rice crop by applying to the locus of the paddy rice crop an effective amount of a compound of Formula I

$JOCH_2Q$ Formula I

wherein

J is

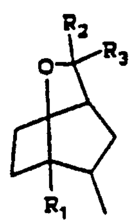

J-1

,

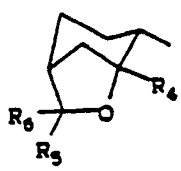

J-2

or

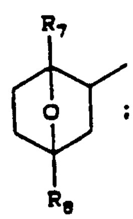

J-3

;

$R_1$ is $CH_3$ or $CH_2CH_3$;
$R_2$ and $R_3$ are independently H, $CH_3$ or $CH_2CH_3$ or
$R_2$ and $R_3$ may be taken together as $-(CH_2)n-$where n is 4 or 5;
$R_4$ is H, $CH_3$ or $CH_2CH_3$;
$R_5$ and $R_6$ are independently $CH_3$ or $CH_2CH_3$;
$R_7$ is $CH_3$ or $CH_2CH_3$;
$R_8$ is $CH_3$, $CH_2CH_3$ or $CH(CH_3)_2$;
Q is

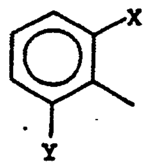 , 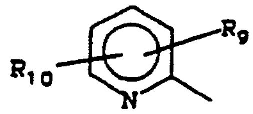 ,

Q-1 Q-2

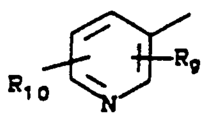 , 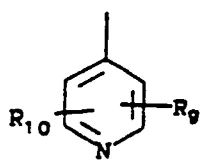 , 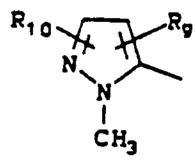 ,

Q-3 Q-4 Q-5

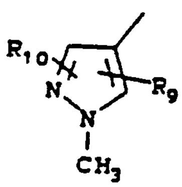 , 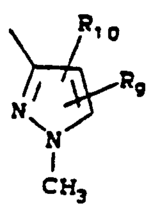 , 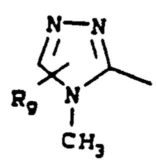 ,

Q-6 , Q-7 Q-8

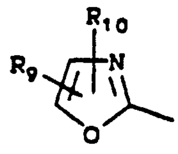
Q-9

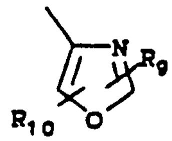
Q-10

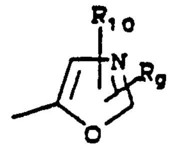
Q-11

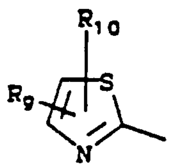
Q-12

,

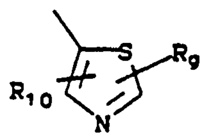
Q-13

or

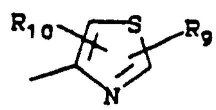
Q-14

X is F, Cl, or $CH_3$;
Y is H, F, or Cl;
$R_9$ is H, F, Cl, Br, $CH_3$ or $CH_2CH_3$; and
$R_{10}$ is H, F, Cl or $CH_3$;
provided that

(1) when J is J-1 or J-3 and Q is Q-2 then $R_9$ and $R_{10}$ are not both H,
(2) when Q is Q-1 then J is J-1, $R_1$ is $CH_3$, and $R_2$ and $R_3$ are both $CH_2CH_3$,
(3) when X is F then Y is other than H,
(4) when X is Cl then Y is other than Cl,
(5) when X is $CH_3$ then Y is H,
(6) when Y is H then X is Cl or $CH_3$,
(7) when Y is F then X is F or Cl, and
(8) when Y is Cl then X is F or H.

2. The method of Claim 1 wherein:
$R_1$ is $CH_3$;
$R_2$ and $R_3$ are $CH_2CH_3$ or $R_2$ and $R_3$ are taken together as $-(CH_2)_n-$ where n is 4 or 5;
$R_4$ is $CH_3$;
$R_5$ and $R_6$ are $CH_3$;
$R_7$ is $CH_3$ or $CH_2CH_3$; and
$R_8$ is $CH_2CH_3$ or $CH(CH_3)_2$.

3. The method of Claim 2 wherein J is J-1.

4. The method of Claim 2 wherein Q is Q-1.

5. The method of Claim 2 wherein the crop is transplanted japonica rice.

6. The method of Claim 2 wherein the crop is transplanted indica rice.

7. The method of Claim 2 wherein among the weeds controlled is barnyardgrass.

8. The method of Claim 1 wherein the compound of Formula I is 6-endo-[(2,6-difluorophenyl)methoxy]-3,3-diethyl-1-methyl-2-oxabicyclo[2.2.2]octane.

9. The method of Claim 1 wherein the compound of Formula I is 6-endo-[(2-chlorophenyl)methoxy]-3,3-diethyl-1-methyl-2-oxabicyclo[2.2.2]octane.

10. The method of Claim 1 wherein the compound of Formula I is 6-endo-[(2-chloro-6-fluorophenyl)-methoxy]-3,3-diethyl-1methyl-2-oxabicyclo[2.2.2.]octane.

11. The method of Claim 1 wherein the compound of Formula I is 3,3-diethyl-1-methyl-6-endo-[(2-methylphenyl)methoxy]-2-oxabicyclo[2.2.2]octane.

12. The method of Claim 1 wherein the compound of Formula I is exo-1-methyl-4-(1-methylethyl)-2-[(4′-thiazolyl)methoxy]-7-oxabicylco[2.2.1]heptane.

13. The method of Claim 1 wherein the compound of Formula I is exo-1,4-diethyl-2-[(4′-thiazolyl)-methoxy]-7-oxabicyclo[2.2.1]heptane.

14. The method of Claim 1 wherein the compound of Formula I is exo-1-methyl-4-(1-methylethyl)-2-[(2′-(6′-fluoropyridyl))methoxy]-7-oxabicyclo[2.2.1]heptane.

15. The method of Claim 1 wherein the compound of Formula I is exo-1,4-diethyl-2-[(2′-(6′-fluoropyridyl))-methoxy]-7-oxabicyclo[2.2.1]heptane.

16. Compounds of Claim 1 of Formula I wherein Q is Q-2 through Q-14, provided that when J is J-1 or J-3 and Q is Q-2 then $R_9$ and $R_{10}$ are not both H.

17. Compounds of Claim 16 of Formula I wherein

$R_1$ is $CH_3$;

$R_2$ and $R_3$ are $CH_2CH_3$ or $R_2$ and $R_3$ are taken together as $-(CH_2)_n-$ where n is 4 or 5;

$R_4$ is $CH_3$;

$R_5$ and $R_6$ are $CH_3$;

$R_7$ is $CH_3$ or $CH_2CH_3$; and

$R_8$ is $CH_2CH_3$ or $CH(CH_3)_2$.

18. Compounds of Claim 17 wherein J is J-3.

19. Compounds of Claim 17 wherein J is J-2.

20. Compounds of Claim 17 wherein J is J-1.

21. The compound of Claim 17 which is exo-1-methyl-4-(1-methylethyl)-2-[(4′-thiazolyl)methoxy]-7-oxabicyclo[2.2.1]heptane.

22. The compound of Claim 14 which is exo-1,4-diethyl--2-[(4′-thiazolyl)methoxy]-7-oxabicyclo[2.2.1]-heptane.

23. The compound of Claim 14 which is exo-1-methyl-4-(1-methylethyl)-2-[(2′-(6′-fluoropyridyl)-)methoxy]-7-oxabicyclo[2.2.1]heptane.

24. The compound of Claim 14 which is exo-1,4-diethyl-2-[(2′-(6′-fluoropyridyl))methoxy]-7-oxabicyclo-[2.2.1]heptane.

25. An agriculturally suitable composition for controlling the growth of undesired vegetation comprising an effective amount of the compound of Claim 16 and at least one of the following:
surfactant, solid diluent or liquid diluent.

26. An agriculturally suitable composition for controlling the growth of undesired vegetation comprising an effective amount of the compound of Claim 17 and at least one of the following:
surfactant, solid diluent or liquid diluent.

27. An agriculturally suitable composition for controlling the growth of undesired vegetation comprising an effective amount of the compound of Claim 18 and at least one of the following:
surfactant, solid diluent or liquid diluent.

28. An agriculturally suitable composition for controlling the growth of undesired vegetation comprising an effective amount of the compound of Claim 21 and at least one of the following:
surfactant, solid diluent or liquid diluent.

29. An agriculturally suitable composition for controlling the growth of undesired vegetation comprising an effective amount of the compound of Claim 22 and at least one of the following:
surfactant, solid diluent or liquid diluent.

30. An agriculturally suitable composition for controlling the growth of undesired vegetation comprising an effective amount of the compound of Claim 23 and at least one of the following:
surfactant, solid diluent or liquid diluent.

31. An agriculturally suitable composition for controlling the growth of undesired vegetation comprising an effective amount of the compound of Claim 24 and at least one of the following:
surfactant, solid diluent or liquid diluent.

European Patent Office

# EUROPEAN SEARCH REPORT

Application Number

EP 90 30 2693

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | US-A-4 798 621 (DU PONT)<br>* Claim 1 * | 1 | C 07 D 493/08<br>C 07 D 405/12<br>C 07 D 413/12<br>C 07 D 417/12<br>A 01 N 43/90<br>A 01 N 43/40<br>A 01 N 43/56<br>A 01 N 43/653<br>A 01 N 43/76<br>A 01 N 43/78 |
| X | CHEMICAL ABSTRACTS, vol. 110, 1989, page 264, abstract no. 110124r, Columbus, Ohio, US; & JP-A-63 165 301 (SHELL KAGAKU K.K.) 08-07-1988<br>* Abstract * | 1 | |
| D,X | US-A-4 486 219 (SHELL)<br>* Claims 1,9 * | 1 | |
| D,X | EP-A-0 081 893 (SHELL)<br>* Claims 1,23 * & US-A-4 670 041 | 16,25 | |
| X | GB-A-2 188 931 (SHELL)<br>* Claims 1,13 * | 16,25 | |
| P,X | EP-A-0 308 170 (DU PONT)(22-03-1989)<br>* Claim 1 * | 1 | TECHNICAL FIELDS SEARCHED (Int. Cl.5)<br>C 07 D 493/00<br>C 07 D 405/00<br>C 07 D 413/00<br>C 07 D 417/00<br>A 01 N 43/00 |

The present search report has been drawn up for all claims

| Place of search | Date of completion of the search | Examiner |
|---|---|---|
| THE HAGUE | 29-05-1990 | ALFARO I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document